(19) Europäisches Patentamt
European Patent Office
Office européen des brevets

(11) Veröffentlichungsnummer: **0 517 052 A1**

(12) **EUROPÄISCHE PATENTANMELDUNG**

(21) Anmeldenummer: **92108733.4**

(22) Anmeldetag: **23.05.92**

(51) Int. Cl.5: **C07D 403/04**, C07D 401/14, C07D 487/14, C07D 415/14, A01N 43/54

(30) Priorität: **07.06.91 DE 4118720**

(43) Veröffentlichungstag der Anmeldung: **09.12.92 Patentblatt 92/50**

(84) Benannte Vertragsstaaten: **BE CH DE DK ES FR GB IT LI NL**

(71) Anmelder: **BAYER AG**

**W-5090 Leverkusen 1 Bayerwerk(DE)**

(72) Erfinder: **Seitz, Thomas, Dr.**
**Mozartstrasse 32**
**W-4019 Monheim(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **Substituierte Imidazolinylpyrimidine.**

(57) Die Erfindung betrifft neue substituierte Imidazolinylpyrimidine der allgemeinen Formel (I)

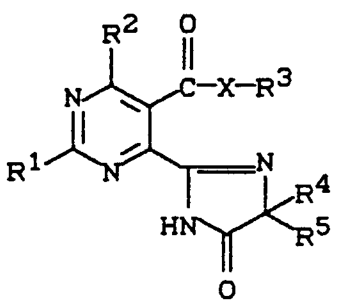

(I)

in welcher

$R^1$ für Wasserstoff, Halogen, Cyano, Nitro oder Thiocyanato oder für einen jeweils gegebenenfalls substituierten Hydroxy-, Mercapto-, Amino-, Carbonyl-, Sulfinyl-, Sulfonyl-, Alkyl-, Alkenyl-, Alkinyl-, Cycloalkyl-, Cycloalkenyl-, Aryl- oder Heterocyclylrest steht,

$R^2$ für Wasserstoff, Hydroxy, Halogen, Alkyl oder Halogenalkyl steht,

$R^3$ für Wasserstoff, für jeweils gegebenenfalls substituiertes Alkyl, Alkenyl, Alkinyl, Cycloalkyl, Cycloalkenyl, Aryl oder Heterocyclyl oder für ein Äquivalent eines anorganischen oder organischen Kations steht,

$R^4$ und $R^5$ unabhängig voneinander jeweils für Wasserstoff, Alkyl, Halogenalkyl oder Cycloalkyl stehen und

X für Sauerstoff oder Schwefel steht,

mehrere Verfahren zu ihrer Herstellung und ihre Verwendung als Herbizide.



Rank Xerox (UK) Business Services

Die Erfindung betrifft neue substituierte Imidazolinylpyrimidine, mehrere Verfahren zu ihrer Herstellung und ihre Verwendung als Herbizide.

Es ist bekannt, daß bestimmte Imidazolinylpyridine wie beispielsweise die Verbindung 2-(4,4-Dimethylimidazolin-5-on-2-yl)-pyridin-3-carbonsäuremethylester herbizide Eigenschaften besitzen (vergleiche z. B. EP 41 623).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue Imidazolinylpyrimidine der allgemeinen Formel (I),

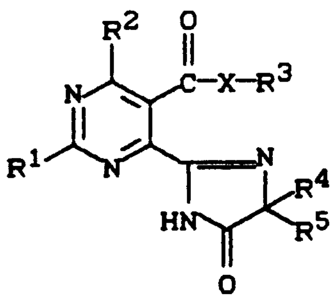

(I)

in welcher

| | |
|---|---|
| $R^1$ | für Wasserstoff, Halogen, Cyano, Nitro oder Thiocyanato oder für einen jeweils gegebenenfalls substituierten Hydroxy-, Mercapto-, Amino-, Carbonyl-, Sulfinyl-, Sulfonyl-, Alkyl-, Alkenyl-, Alkinyl-, Cycloalkyl-, Cycloalkenyl-, Aryl- oder Heterocyclylrest steht, |
| $R^2$ | für Wasserstoff, Hydroxy, Halogen, Alkyl oder Halogenalkyl steht, |
| $R^3$ | für Wasserstoff, für jeweils gegebenenfalls substituiertes Alkyl, Alkenyl, Alkinyl, Cycloalkyl, Cycloalkenyl, Aryl oder Heterocyclyl oder für ein Äquivalent eines anorganischen oder organischen Kations steht, |
| $R^4$ und $R^5$ | unabhängig voneinander jeweils für Wasserstoff, Alkyl, Halogenalkyl oder Cycloalkyl stehen und |
| X | für Sauerstoff oder Schwefel steht, |

gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen Imidazolinylpyrimidine der allgemeinen Formel (I),

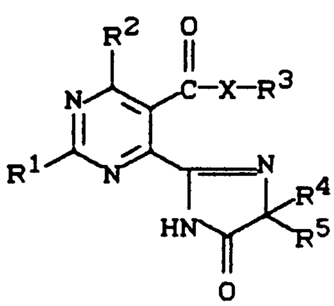

(I)

in welcher

| | |
|---|---|
| $R^1$ | für Wasserstoff, Halogen, Cyano, Nitro oder Thiocyanato oder für einen jeweils gegebenenfalls substituierten Hydroxy-, Mercapto-, Amino-, Carbonyl-, Sulfinyl-, Sulfonyl-, Alkyl-, Alkenyl-, Alkinyl-, Cycloalkyl-, Cycloalkenyl-, Aryl- oder Heterocyclylrest steht, |
| $R^2$ | für Wasserstoff, Hydroxy, Halogen, Alkyl oder Halogenalkyl steht, |
| $R^3$ | für Wasserstoff, für jeweils gegebenenfalls substituiertes Alkyl, Alkenyl, Alkinyl, Cycloalkyl, |

Cycloalkenyl, Aryl oder Heterocyclyl oder für ein Äquivalent eines anorganischen oder organischen Kations steht,

$R^4$ und $R^5$ unabhängig voneinander jeweils für Wasserstoff, Alkyl, Halogenalkyl oder Cycloalkyl stehen und

X für Sauerstoff oder Schwefel steht,

erhält, wenn man

(a) Pyrimidin-4,5-dicarbonsäurediester der Formel (II),

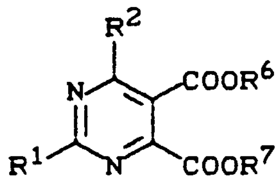

(II)

in welcher

$R^1$ und $R^2$ die oben angegebene Bedeutung haben und

$R^6$ und $R^7$ unabhängig voneinander jeweils für Alkyl stehen,

mit α-Aminosäureamiden der Formel (III),

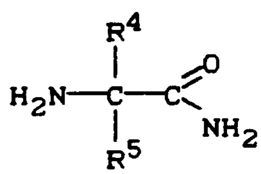

(III)

in welcher

$R^4$ und $R^5$ die oben angegebene Bedeutung haben,

gegebenenfalls in Gegenwart eines Verdünnungsmittels und gegebenenfalls in Gegenwart eines Reaktionshilfsmittels umsetzt und gegebenenfalls

(b) in einer anschließenden Folgereaktion die so erhältlichen Imidazolinylpyrimidincarbonsäuren der Formel (Ia),

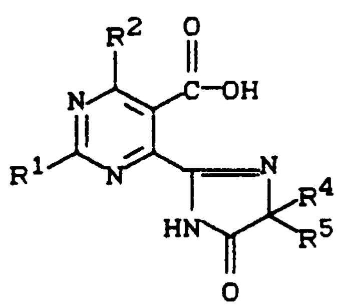

(Ia)

in welcher

$R^1$, $R^2$, $R^4$ und $R^5$ die oben angegebene Bedeutung haben,

zunächst in einer 1. Stufe mit einem Kondensationsmittel gegebenenfalls in Gegenwart eines Verdünnungsmittels und gegebenenfalls in Gegenwart eines Reaktionshilfsmittels umsetzt und anschließend die so erhältlichen Imidazo-pyrrolo-pyrimidine der Formeln (IVa) und (IVb),

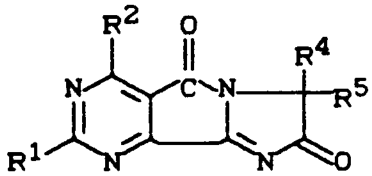

(IVa)

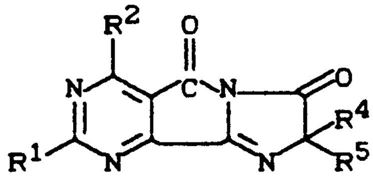

(IVb)

in welcher

$R^1$, $R^2$, $R^4$ und $R^5$ die oben angegebene Bedeutung haben,

in einer anschließenden 2. Stufe mit Alkoholen oder Thiolen der Formel (V),

$R^3$—X—H (V)

in welcher

$R^3$ und X die oben angegebene Bedeutung haben,

gegebenenfalls in Gegenwart eines Verdünnungsmittels und gegebenenfalls in Gegenwart eines Reaktionshilfsmittels umsetzt.

Schließlich wurde gefunden, daß die neuen Imidazolinylpyrimidine der allgemeinen Formel (I) ebenso wie die als Zwischenprodukte benannten Imidazo-pyrrolo-pyrimidin der allgemeinen Formeln (IVa) und (IVb) herbizide Eigenschaften besitzen.

Überraschenderweise zeigen die erfindungsgemäßen Imidazolinylpyrimidine der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten Imidazolinylpyridinen, wie beispielsweise die Verbindung 2-(4,4-Dimethylimidazolin-5-on-2-yl)-pyridin-3-carbonsäuremethylester, welche chemisch und wirkungsmäßig naheliegende Verbindungen sind.

Die erfindungsgemäßen Imidazolinylpyrimidine sind durch die Formel (I) allgemein definiert. Bevorzugt sind Verbindungen der Formel (I), bei welchen

$R^1$ für Wasserstoff, Fluor, Chlor, Brom, Iod, Cyano, Nitro oder Thiocyanato steht;
außerdem für jeweils gegebenenfalls substituiertes Hydroxy oder Mercapto steht, wobei als Substituenten jeweils infrage kommen: geradkettiges oder verzweigtes Alkyl mit 1 bis 8 Kohlenstoffatomen, geradkettiges oder verzweigtes Halogenalkyl mit 1 bis 8 Kohlenstoffatomen und 1 bis 17 gleichen oder verschiedenen Halogenatomen, geradkettiges oder verzweigtes Alkoxyalkyl mit jeweils 1 bis 8 Kohlenstoffatomen in den einzelnen Alkylteilen, geradkettiges oder verzweigtes Dialkylaminoalkyl mit jeweils 1 bis 8 Kohlenstoffatomen in den einzelnen Alkylteilen, geradkettiges oder verzweigtes Alkanoyl mit 1 bis 9 Kohlenstoffatomen, geradkettiges oder verzweigtes Alkoxycarbonyl mit 2 bis 9 Kohlenstoffatomen, geradkettiges oder verzweigtes Alkylsulfonyl mit 1 bis 8 Kohlenstoffatomen, geradkettiges oder verzweigtes Alkoxysulfonyl mit 1 bis 8 Kohlenstoffatomen, jeweils gegebenenfalls im Aryl- bzw. Heterocyclylteil einfach oder mehrfach, gleich oder verschieden substituiertes Arylalkyl mit 1 bis 8 Kohlenstoffatomen im geradkettigen oder verzweigten Alkylteil und 6 bis 10 Kohlenstoffatomen im Arylteil, Aryl mit 6 bis 10 Kohlenstoffatomen, Arylcarbonyl, Arylsulfonyl oder Aryloxysulfonyl mit jeweils 6 bis 10 Kohlenstoffatomen im Arylteil, Heterocyclyl oder Heterocyclylalkyl mit jeweils 2 bis 9 Kohlenstoffatomen und 1 bis 5 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

außerdem für gegebenenfalls einfach oder zweifach, gleich oder verschieden substituiertes Amino steht, wobei als Substituenten infrage kommen:

geradkettiges oder verzweigtes Alkyl mit 1 bis 8 Kohlenstoffatomen, geradkettiges oder verzweigtes Alkenyl mit 2 bis 8 Kohlenstoffatomen, geradkettiges oder verzweigtes Alkinyl mit 2 bis 8 Kohlenstoffatomen, geradkettiges oder verzweigtes Halogenalkyl mit 1 bis 8 Kohlenstoffatomen und 1 bis 17 gleichen oder verschiedenen Halogenatomen, geradkettiges oder verzweigtes Alkoxyalkyl mit jeweils 1 bis 8 Kohlenstoffatomen in den einzelnen Alkylteilen geradkettiges oder verzweigtes Dialkylaminoalkyl mit jeweils 1 bis 8 Kohlenstoffatomen in den einzelnen Alkylteilen, geradkettiges oder verzweigtes Alkanoyl mit 1 bis 9 Kohlenstoffatomen, geradkettiges oder verzweigtes Alkoxycarbonyl mit 2 bis 9 Kohlenstoffatomen, geradkettiges oder verzweigtes Alkylsulfonyl mit 1 bis 8 Kohlenstoffatomen, geradkettiges oder verzweigtes Alkoxysulfonyl mit 1 bis 8 Kohlenstoffatomen, geradkettiges oder verzweigtes Dialkylaminosulfonyl mit jeweils 1 bis 8 Kohlenstoffatomen in den einzelnen Alkylteilen, jeweils gegebenenfalls im Aryl- bzw. Heterocyclylteil einfach oder mehrfach, gleich oder verschieden substituiertes Arylalkyl mit 1 bis 8 Kohlenstoffatomen im geradkettigen oder verzweigten Alkylteil und 6 bis 10 Kohlenstoffatomen im Arylteil, Aryl mit 6 bis 10 Kohlenstoffatomen, Arylcarbonyl, Arylsulfonyl oder Aryloxysulfonyl mit jeweils 6 bis 10 Kohlenstoffatomen im Arylteil, Heterocyclyl oder Heterocyclylalkyl mit jeweils 2 bis 9 Kohlenstoffatomen und 1 bis 5 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bsw. Heterocyclylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

außerdem für eine jeweils substituierte Carbonyl-,Sulfinyl- oder Sulfonylgruppe steht, wobei als Substituenten jeweils infrage kommen:

Wasserstoff, Hydroxy, Amino, jeweils geradkettiges oder verzweigtes Alkyl, Alkoxy, Alkylamino oder Dialkylamino mit jeweils 1 bis 8 Kohlenstoffatomen in den einzelnen Alkylteilen, jeweils geradkettiges oder verzweigtes Halogenalkyl oder Halogenalkoxy mit jeweils 1 bis 8 Kohlenstoffatomen und 1 bis 17 gleichen oder verschiedenen Halogenatomen, jeweils gegebenenfalls im Arylteil einfach oder mehrfach, gleich oder verschieden substituiertes Aryl, Aryloxy, Arylamino oder Diarylamino mit jeweils 6 bis 10 Kohlenstoffatomen in den einzelnen Arylteilen, wobei als Substituenten in den einzelnen Arylteilen jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

außerdem für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:

Fluor, Chlor, Brom, Iod, Hydroxy, Cyano, Nitro, Aminocarbonyl, Formamido, jeweils

geradkettiges oder verzweigtes Alkoxy, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 8 Kohlenstoffatomen in den einzelnen Alkylteilen und gegebenenfalls 1 bis 17 gleichen oder verschiedenen Halogenatomen, jeweils gegebenenfalls im Aryl- oder Heterocyclylteil einfach oder mehrfach, gleich oder verschieden substituiertes Aryl, Aryloxy, Arylthio, Arylamino, Diarylamino, Arylcarbonyl oder Arylsulfonyl mit jeweils 6 bis 10 Kohlenstoffatomen in den einzelnen Arylteilen oder Heterocyclyl mit 2 bis 9 Kohlenstoffatomen und 1 bis 5 gleichen oder verschiedenen Heteroatomen, insbesondere Stickstoff, Sauerstoff und/oder Schwefel, wobei als Aryl- oder Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

außerdem für gegebenenfalls einfach oder mehrfach, gleich oder verschieden substituiertes, geradkettiges oder verzweigtes <u>Alkenyl</u> mit 2 bis 8 Kohlenstoffatomen steht, wobei als Substituenten infrage kommen:

Fluor, Chlor, Brom, Iod oder Cyano;

außerdem für gegebenenfalls einfach oder mehrfach, gleich oder verschieden substituiertes, geradkettiges oder versweigtes <u>Alkinyl</u> mit 2 bis 8 Kohlenstoffatomen steht, wobei als Substituenten infrage kommen:

Fluor, Chlor, Brom oder Iod;

außerdem für jeweils gegebenenfalls einfach oder mehrfach, gleich oder verschieden substituiertes <u>Cycloalkyl</u> oder <u>Cycloalkenyl</u> mit jeweils 3 bis 8 Kohlenstoffatomen steht, wobei als Substituenten jeweils infrage kommen:

Fluor, Chlor, Brom, Iod sowie jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen;

außerdem für jeweils gegebenenfalls einfach oder mehrfach, gleich oder verschieden substituiertes <u>Aryl</u> mit 6 bis 10 Kohlenstoffatomen oder <u>Heterocyclyl</u> mit 2 bis 9 Kohlenstoffatomen und 1 bis 5 gleichen oder verschiedenen Heteroatomen, insbesondere Stickstoff, Sauerstoff und/oder Schwefel steht, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^2$ für Wasserstoff, Hydroxy, Fluor, Chlor, Brom, Iod, für geradkettiges oder verzweigtes Alkyl mit 1 bis 8 Kohlenstoffatomen oder für geradkettiges oder verzweigtes Halogenalkyl mit 1 bis 8 Kohlenstoffatomen und 1 bis 17 gleichen oder verschiedenen Halogenatomen steht,

$R^3$ für Wasserstoff oder für ein Äquivalent eines Alkali-, Erdalkali- oder eines gegebenenfalls einfach oder mehrfach, gleich oder verschieden durch geradkettiges oder verzweigtes Alkyl mit 1 bis 18 Kohstoffatomen und/oder Benzyl substituierten Ammoniumkations steht,

außerdem für gegebenenfalls einfach oder mehrfach, gleich oder verschieden substituiertes, geradkettiges oder verzweigtes <u>Alkyl</u> mit 1 bis 18 Kohlenstoffatomen steht, wobei als Substituenten infrage kommmen:

Fluor, Chlor, Brom, Iod, Hydroxy, Cyano, Nitro, Aminocarbonyl, Formamido, jeweils geradkettiges oder verzweigtes Alkoxy, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 8 Kohlenstoffatomen in den einzelnen Alkylteilen

und gegebenenfalls 1 bis 17 gleichen oder verschiedenen Halogenatomen, jeweils gegebenenfalls im Aryl- oder Heterocyclylteil einfach oder mehrfach, gleich oder verschieden substituiertes Aryl, Aryloxy, Arylthio, Arylamino, Diarylamino, Arylcarbonyl oder Arylsulfonyl mit jeweils 6 bis 10 Kohlenstoffatomen in den einzelnen Arylteilen oder Heterocyclyl mit 2 bis 9 Kohlenstoffatomen und 1 bis 5 gleichen oder verschiedenen Heteroatomen, insbesondere Stickstoff, Sauerstoff und/oder Schwefel, wobei als Aryl- oder Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

außerdem für gegebenenfalls einfach oder mehrfach, gleich oder verschieden substituiertes, geradkettiges oder verzweigtes Alkenyl mit 2 bis 8 Kohlenstoffatomen steht, wobei als Substituenten infrage kommen:

Fluor, Chlor, Brom, Iod, Cyano oder gegebenenfalls einfach oder mehrfach, gleich oder verschieden substituiertes Aryl mit 6 bis 10 Kohlenstoffatomen, wobei als Substituenten infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;

außerdem für gegebenenfalls einfach oder mehrfach, gleich oder verschieden substituiertes, geradkettiges oder verzweigtes Alkinyl mit 2 bis 8 Kohlenstoffatomen steht, wobei als Substituenten infrage kommen:

Fluor, Chlor, Brom oder Iod;

außerdem für jeweils gegebenenfalls einfach oder mehrfach, gleich oder verschieden substituiertes Cycloalkyl oder Cycloalkenyl mit jeweils 3 bis 8 Kohlenstoffatomen steht, wobei als Substituenten jeweils infrage kommen:

Fluor, Chlor, Brom, Iod sowie jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen;

außerdem für jeweils gegebenenfalls einfach oder mehrfach, gleich oder verschieden substituiertes Aryl mit 6 bis 10 Kohlenstoffatomen oder Heterocyclyl mit 2 bis 9 Kohlenstoffatomen und 1 bis 5 gleichen oder verschiedenen Heteroatomen, insbesondere Stickstoff, Sauerstoff und/oder Schwefel steht, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^4$ und $R^5$ unabhängig voneinander jeweils für Wasserstoff, für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 17 gleichen oder verschiedenen Halogenatomen oder für Cycloalkyl mit 3 bis 8 Kohlenstoffatomen stehen und

X für Sauerstoff oder Schwefel steht.

Besonders bevorzugt sind Verbindungen der Formel (I), bei welchen

$R^1$ für Wasserstoff, Fluor, Chlor, Brom, Iod, Cyano, Nitro oder Thiocyanato steht;

außerdem für jeweils gegebenenfalls substituiertes Hydroxy oder Mercapto steht, wobei als Substituenten jeweils infrage kommen:

geradkettiges oder verzweigtes Alkyl mit 1 bis 4 Kohlenstoffatomen, geradkettiges oder

verzweigtes Halogenalkyl mit 1 bis 4 Kohlenstoffatomen und 1 bis 9 gleichen oder verschiedenen Halogenatomen, geradkettiges oder verzweigtes Alkoxyalkyl mit jeweils 1 bis 4 Kohlenstoffatomen in den einzelnen Alkylteilen, geradkettiges oder verzweigtes Dialkylaminoalkyl mit jeweils 1 bis 4 Kohlenstoffatomen in den einzelnen Alkylteilen, geradkettiges oder verzweigtes Alkanoyl mit 1 bis 5 Kohlenstoffatomen, geradkettiges oder verzweigtes Alkoxycarbonyl mit 2 bis 5 Kohlenstoffatomen, geradkettiges oder verzweigtes Alkylsulfonyl mit 1 bis 4 Kohlenstoffatomen, geradkettiges oder verzweigtes Alkoxysulfonyl mit 1 bis 4 Kohlenstoffatomen, jeweils gegebenenfalls im Aryl- bzw. Heterocyclylteil ein- bis fünffach, gleich oder verschieden substituiertes Arylalkyl mit 1 bis 4 Kohlenstoffatomen im geradkettigen oder verzweigten Alkylteil und 6 oder 10 Kohlenstoffatomen im Arylteil, Aryl mit 6 oder 10 Kohlenstoffatomen, Arylcarbonyl, Arylsulfonyl oder Aryloxysulfonyl mit jeweils 6 oder 10 Kohlenstoffatomen im Arylteil, Heterocyclyl oder Heterocyclylalkyl mit jeweils 2 bis 9 Kohlenstoffatomen und 1 bis 4 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;

außerdem für gegebenenfalls einfach oder zweifach, gleich oder verschieden substituiertes Amino steht, wobei als Substituenten infrage kommen:

geradkettiges oder verzweigtes Alkyl mit 1 bis 4 Kohlenstoffatomen, geradkettiges oder verzweigtes Alkenyl mit 2 bis 5 Kohlenstoffatomen, geradkettiges oder verzweigtes Alkinyl mit 2 bis 5 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxyalkyl mit jeweils 1 bis 4 Kohlenstoffatomen in den einzelnen Alkylteilen, geradkettiges oder verzweigtes Dialkylaminoalkyl mit jeweils 1 bis 4 Kohlenstoffatomen in den einzelnen Alkylteilen, geradkettiges oder verzweigtes Alkanoyl mit 1 bis 5 Kohlenstoffatomen, geradkettiges oder verzweigtes Alkoxycarbonyl mit 2 bis 5 Kohlenstoffatomen, geradkettiges oder verzweigtes Alkylsulfonyl mit 1 bis 4 Kohlenstoffatomen, geradkettiges oder verzweigtes Alkoxysulfonyl mit 1 bis 4 Kohlenstoffatomen, geradkettiges oder verzweigtes Dialkylaminosulfonyl mit jeweils 1 bis 4 Kohlenstoffatomen in den einzelnen Alkylteilen, jeweils gegebenenfalls im Aryl- bzw. Heterocyclylteil ein- bis fünffach, gleich oder verschieden substituiertes Arylalkyl mit 1 bis 4 Kohlenstoffatomen im geradkettigen oder verzweigten Alkylteil und 6 oder 10 Kohlenstoffatomen im Arylteil, Aryl mit 6 oder 10 Kohlenstoffatomen, Arylcarbonyl, Arylsulfonyl oder Aryloxysulfonyl mit jeweils 6 oder 10 Kohlenstoffatomen im Arylteil, Heterocyclyl oder Heterocyclylalkyl mit jeweils 2 bis 9 Kohlenstoffatomen und 1 bis 4 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;

außerdem für eine jeweils substituierte Carbonyl-, Sulfinyl- oder Sulfonylgruppe steht, wobei als Substituenten jeweils infrage kommen:

Wasserstoff, Hydroxy, Amino, jeweils geradkettiges oder verzweigtes Alkyl, Alkoxy, Alkylamino oder Dialkylamino mit jeweils 1 bis 4 Kohlenstoffatomen in den einzelnen Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen, jeweils gegebenenfalls im Arylteil ein- bis fünffach, gleich oder verschieden substituiertes Aryl, Aryloxy, Arylamino oder Diarylamino mit jeweils 6 oder 10 Kohlenstoffatomen in den einzelnen Arylteilen, wobei als Substituenten in den einzelnen Arylteilen jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;

außerdem für gegebenenfalls einfach bis fünffach, gleich oder verschieden substituiertes, geradkettiges oder verzweigtes Alkyl mit 1 bis 6 Kohlenstoffatomen steht, wobei als Substituenten infrage kommen:

Fluor, Chlor, Brom, Iod, Hydroxy, Cyano, Nitro, Aminocarbonyl, Formamido, jeweils geradkettiges oder verzweigtes Alkoxy, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 4 Kohlenatoffatomen in den einzelnen Alkylteilen und gegebenenfalls 1 bis 9 gleichen oder verschiedenen Halogenatomen, jeweils gegebenenfalls im Aryl- oder Heterocyclylteil ein- bis fünffach, gleich oder verschieden substituiertes Aryl, Aryloxy, Arylthio, Arylamino, Diarylamino, Arylcarbonyl oder Arylsulfonyl mit jeweils 6 oder 10 Kohlenstoffatomen in den einzelnen Arylteilen oder Heterocyclyl mit 2 bis 9 Kohlenatoffatomen und 1 bis 4 gleichen oder verschiedenen Heteroatomen, insbesondere Stickstoff, Sauerstoff und/oder Schwefel, wobei als Aryl- oder Heterocyclylsubstituenten jeweils infrage kommen:

Halogen, Cyano, Nitro, jeweils geradkattiges oder verzweigtes Alkyl, Alkoxy oder Alkylthio mit jeweils 1 bis 4 Kohlenstoffatomen, jeweils garadkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettigas oder verzweigtes Alkyl mit 1 bis 4 Kohlenatoffatomen substituiertes Phenyl;

außerdem für gegebenenfalls einfach bis dreifach, gleich oder verschieden substituiertes, geradkettiges oder verzweigtes Alkenyl mit 2 bis 5 Kohlenstoffatomen steht, wobei als Substituenten infrage kommen:

Fluor, Chlor, Brom, Iod oder Cyano;

außerdem für gegebenenfalls einfach bis dreifach, gleich oder verschieden substituiertes, geradkettiges oder verzweigtes Alkinyl mit 2 bis 5 Kohlenstoffatomen steht, wobei als Substituenten infrage kommen:

Fluor, Chlor, Brom oder Iod;

außerdem für jeweils gegebenenfalls einfach bis fünffach, gleich oder verschieden substituiertes Cycloalkyl oder Cycloalkenyl mit jeweils 3 bis 6 Kohlenstoffatomen steht, wobei als Substituenten jeweils infrage kommen:

Fluor, Chlor, Brom, Iod sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen;

außerdem für jeweils gegebenenfalls einfach bis fünffach, gleich oder verschieden substituiertes Aryl mit 6 oder 10 Kohlenstoffatomen oder Heterocyclyl mit 2 bis 9 Kohlenstoffatomen und 1 bis 4 gleichen oder verschiedenen Heteroatomen, insbesondere Stickstoff, Sauerstoff und/oder Schwefel steht, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;

$R^2$ für Wasserstoff, Hydroxy, Fluor, Chlor, Brom, Iod, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^3$ für Wasserstoff oder für ein Äquivalent eines Alkali-, Erdalkali- oder eines gegebenenfalls einfach bis vierfach, gleich oder verschieden durch geradkettiges oder verzweigtes Alkyl mit 1 bis 18 Kohlenstoffatomen und/oder Benzyl substituierten Ammoniumkations steht, außerdem für gegebenenfalls einfach bis fünffach, gleich oder verschieden substituiertes, geradkettiges oder verzweigtes Alkyl mit 1 bis 12 Kohlenstoffatomen steht, wobei als Substituenten infrage kommen:

Fluor, Chlor, Brom, Iod, Hydroxy, Cyano, Nitro, Aminocarbonyl, Formamido, jeweils geradkettiges oder verzweigtes Alkoxy, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 8 Kohlenstoffatomen in den einzelnen Alkylteilen und gegebenenfalls 1 bis 17 gleichen oder verschiedenen Halogenatomen, jeweils gegebenenfalls im Aryl- oder Heterocyclylteil einfach bis fünffach, gleich oder verschieden substituiertes Aryl, Aryloxy, Arylthio, Arylamino, Diarylamino, Arylcarbonyl oder Arylsulfonyl mit jeweils 6 oder 10 Kohlenstoffatomen in den einzelnen Arylteilen oder Heterocyclyl mit 2 bis 9 Kohlenstoffatomen und 1 bis 4 gleichen oder verschiedenen Heteroatomen, insbesondere Stickstoff, Sauerstoff und/oder Schwefel, wobei als Aryl- oder Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;

außerdem für gegebenenfalls einfach bis dreifach, gleich oder verschieden substituiertes, geradkettiges oder verzweigtes Alkenyl mit 2 bis 5 Kohlenstoffatomen steht, wobei als Substituenten infrage kommen:

Fluor, Chlor, Brom, Iod, Cyano oder gegebenenfalls ein- bis dreifach, gleich oder verschieden substituiertes Aryl mit 6 oder 10 Kohlenstoffatomen, wobei als Substituenten infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;

außerdem für gegebenenfalls einfach bis dreifach, gleich oder verschieden substituiertes, geradkettiges oder verzweigtes Alkinyl mit 2 bis 5 Kohlenstoffatomen steht, wobei als Substituenten infrage kommen:

Fluor, Chlor, Brom oder Iod;

außerdem für jeweils gegebenenfalls einfach bis fünffach, gleich oder verschieden substituiertes Cycloalkyl oder Cycloalkenyl mit jeweils 3 bis 6 Kohlenstoffatomen steht, wobei als Substituenten jeweils infrage kommen:

Fluor, Chlor, Brom, Iod sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen;

außerdem für jeweils gegebenenfalls einfach bis fünffach, gleich oder verschieden substituiertes Aryl mit 6 oder 10 Kohlenstoffatomen oder Heterocyclyl mit 2 bis 9 Kohlenstoffatomen und 1 bis 4 gleichen oder verschiedenen Heteroatomen, insbesondere Stickstoff, Sauerstoff und/oder Schwefel steht, wobei als Aryl- bzw. Heterocyclylsubstituenten jeweils

infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;

$R^4$ und $R^5$ unabhängig voneinander jeweils für Wasserstoff, für geradkettiges oder verzweigtes Alkyl mit 1 bis 4 Kohlenstoffatomen, für geradkettiges oder verzweigtes Halogenalkyl mit 1 bis 4 Kohlenstoffatomen und 1 bis 9 gleichen oder verschiedenen Halogenatomen oder für Cycloalkyl mit 3 bis 6 Kohlenstoffatomen stehen und

X für Sauerstoff oder Schwefel steht.

Insbesondere bevorzugt sind Verbindungen der Formel (I), bei welchen

$R^1$ für Wasserstoff steht;
außerdem für jeweils gegebenenfalls substituiertes Hydroxy oder Mercapto steht, wobei als Substituenten jeweils infrage kommen:
geradkettiges oder verzweigtes Alkyl mit 1 bis 4 Kohlenstoffatomen, geradkettiges oder verzweigtes Alkoxyalkyl mit jeweils 1 bis 4 Kohlenstoffatomen in den einzelnen Alkylteilen, geradkettiges oder verzweigtes Dialkylaminoalkyl mit jeweils 1 bis 4 Kohlenstoffatomen in den einzelnen Alkylteilen, geradkettiges oder verzweigtes Halogenalkyl mit 1 bis 4 Kohlenstoffatomen und 1 bis 9 gleichen oder verschiedenen Halogenatomen oder jeweils gegebenenfalls im Phenyl- bzw. Heteroarylteil ein- bis dreifach, gleich oder verschieden substituiertes Phenylalkyl mit 1 bis 4 Kohlenstoffatomen im geradkettigen oder verzweigten Alkylteil, Phenyl, Pyridyl, Pyridylmethyl, Furanylmethyl, Thienylmethyl, Thiazolylmethyl oder Triazolylmethyl, wobei als Phenyl- bzw. Heteroarylsubstituenten jeweils infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für gegebenenfalls einfach oder zweifach, gleich oder verschieden substituiertes Amino steht, wobei als Substituenten infrage kommen:
geradkettiges oder verzweigtes Alkyl mit 1 bis 4 Kohlenstoffatomen, Allyl, Propargyl, geradkettiges oder verzweigtes Alkoxyalkyl mit jeweils 1 bis 4 Kohlenstoffatomen in den einzelnen Alkylteilen, geradkettiges oder verzweigtes Dialkylaminoalkyl mit jeweils 1 bis 4 Kohlenstoffatomen in den einzelnen Alkylteilen, geradkettiges oder verzweigtes Alkanoyl mit 1 bis 5 Kohlenstoffatomen, geradkettiges oder verzweigtes Alkoxycarbonyl mit 2 bis 5 Kohlenstoffatomen, jeweils geradkettiges oder verzweigtes Alkylsulfinyl oder Alkylsulfonyl mit jeweils 1 bis 4 Kohlenstoffatomen;
außerdem für eine jeweils substituierte Carbonyl-,Sulfinyl- oder Sulfonylgruppe steht, wobei als Substituenten jeweils infrage kommen:
jeweils geradkettiges oder verzweigtes Alkyl, Alkoxy, Alkylamino oder Dialkylamino mit jeweils 1 bis 4 Kohlenstoffatomen in den einzelnen Alkylteilen;
außerdem für gegebenenfalls ein- bis dreifach, gleich oder verschieden substituiertes, geradkettiges oder verzweigtes Alkyl mit 1 bis 6 Kohlenstoffatomen steht, wobei als Substituenten infrage kommen:
Fluor, Chlor, jeweils geradkettiges oder verzweigtes Alkoxy oder Alkylthio mit jeweils 1 bis 4 Kohlenstoffatomen, jeweils geradkettiges oder verzweigtes Halogenalkoxy oder Halogenalkylthio mit jeweils 1 bis 4 Kohlenstoffatomen und 1 bis 9 Halogenatomen oder gegebenenfalls ein- bis dreifach, gleich oder verschieden substituiertes Phenyl, wobei als Substituenten infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für geradkettiges oder verzweigtes Alkenyl mit 3 bis 6 Kohlenstoffatomen steht;
außerdem für geradkettiges oder verzweigtes Alkinyl mit 2 bis 6 Kohlenstoffatomen steht;
außerdem für gegebenenfalls ein- bis dreifach, gleich oder verschieden durch Methyl und/oder

Chlor substituiertes Cyclopropyl steht;
außerdem für gegebenenfalls ein- bis dreifach, gleich oder verschieden substituiertes Phenyl steht, wobei als Substituenten jeweils infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für jeweils gegebenenfalls ein- bis dreifach, gleich oder verschieden substituiertes Pyridyl, Furanyl, Tetrahydrofuranyl, Thienyl oder Thiazolyl steht, wobei als Substituenten jeweils infrage kommen:
jeweils geradkettiges oder verzweigtes Alkyl oder Alkoxy mit jeweils 1 bis 4 Kohlenstoffatomen;

$R^2$ für Wasserstoff oder Methyl steht,

$R^3$ für Wasserstoff oder für ein Äquivalent eines Alkali-, Erdalkali- oder eines gegebenenfalls einfach bis vierfach, gleich oder verschieden durch geradkettiges oder verzweigtes Alkyl mit 1 bis 18 Kohlenstoffatomen und/oder Benzyl substituierten Ammoniumkations steht;
außerdem für gegebenenfalls ein- bis dreifach, gleich oder verschieden substituiertes, geradkettiges oder verzweigtes Alkyl mit 1 bis 12 Kohlenstoffatomen steht, wobei als Substituenten infrage kommen:
Fluor, Chlor, Hydroxy, Cyano, jeweils geradkettiges oder verzweigtes Alkoxy, Alkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 8 Kohlenstoffatomen in den einzelnen Alkylteilen, jeweils geradkettiges oder verzweigtes Halogenalkoxy oder Halogenalkylthio mit jeweils 1 bis 8 Kohlenstoffatomen und 1 bis 17 Halogenatomen sowie jeweils gegebenenfalls im Phenyl- oder Heteroarylteil einfach bis dreifach, gleich oder verschieden substituiertes Phenyl, Phenoxy, Phenylthio, Phenylamino, Phenylcarbonyl, Phenylsulfonyl, Pyridyl, Furanyl, Tetrahydrofuranyl, Thienyl oder Thiazolyl, wobei als Phenyl- oder Heteroarylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen;
außerdem für geradkettiges oder verzweigtes Alkenyl mit 2 bis 6 Kohlenstoffatomen steht;
außerdem für geradkettiges oder verzweigtes Alkinyl mit 2 bis 6 Kohlenstoffatomen steht;
außerdem für jeweils gegebenenfalls ein- bis dreifach, gleich oder verschieden durch Fluor, Chlor und/oder Methyl substituiertes Cyclopentyl oder Cyclohexyl steht;
außerdem für gegebenenfalls ein- bis dreifach, gleich oder verschieden substituiertes Phenyl steht, wobei als Substituenten jeweils infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für jeweils gegebenenfalls ein- bis dreifach, gleich oder verschieden substituiertes Pyridyl, Furanyl, Tetrahydrofuranyl, Thienyl oder Thiazolyl steht, wobei als Substituenten jeweils infrage kommen:
jeweils geradkettiges oder verzweigtes Alkyl oder Alkoxy mit jeweils 1 bis 4 Kohlenstoffatomen;

$R^4$ für Wasserstoff, Methyl oder Fluormethyl steht,

$R^5$ für Methyl, Ethyl, n- oder i-Propyl oder Cyclopropyl steht und

X für Sauerstoff oder Schwefel steht.

Ganz besonders bevorzugt sind Verbindungen der Formel (I), bei welchen

$R^1$ für Wasserstoff, Hydroxy, Methoxy, Ethoxy, n- oder i-Propoxy, n-, i-, s- oder t-Butoxy, Trifluormethoxy, Mercapto, Methylthio, Ethylthio, n- oder i-Propylthio, n-, i-, s- oder t-Butylthio, Trifluormethylthio, Benzylthio, 2-Pyridylthio, Amino, N-Methylamino, N-Ethylamino, N-n-Propylamino, N-i-Propylamino, N,N-Dimethylamino, N,N-Diethylamino, N-Methyl-N-Ethylamino, Methylcarbonylamino, Ethylcarbonylamino, Methoxycarbonylamino, Ethoxycarbonylamino, Methylsulfinyl, Ethylsulfinyl, Methylsulfonyl, Ethylsulfonyl, Methyl, Ethyl, n- oder i-Propyl, n-, i-, s- oder t-Butyl, n- oder i-Pentyl, n- oder i-Hexyl, Fluormethyl, Trifluormethyl, Methoxymethyl, Ethoxymethyl, Methylthiomethyl, Ethylthiomethyl, Allyl, Ethinyl, Propargyl, Cyclopropyl oder für gegebenenfalls einfach oder zweifach, gleich oder verschieden substituiertes Phenyl steht, wobei als Substituenten infrage kommen:

Fluor, Chlor, Brom, Methyl, Ethyl, n- oder i-Propyl, n-, i-, s- oder t-Butyl, Methoxy, Ethoxy, n-oder i-Propoxy, n-, i-, s- oder t-Butoxy, Methylthio, Ethylthio, Trifluormethyl, Trifluormethoxy oder Trifluormethylthio;

außerdem für jeweils gegebenenfalls einfach oder zweifach, gleich oder verschieden substituiertes Pyridyl, Furanyl, Thienyl oder Thiazolyl steht, wobei als Substituenten jeweils infrage kommen: Methyl, Ethyl, n- oder i-Propyl, n-, i-, s- oder t-Butyl, Methoxy, Ethoxy, n- oder i-Propoxy, n-, i-, s- oder t-Butoxy,

$R^2$ für Wasserstoff steht,

$R^3$ für Wasserstoff, für ein Natrium-, Kalium-, Calcium- oder für ein gegebenenfalls ein- bis vierfach, gleich oder verschieden durch Methyl, Ethyl, n- oder i-Propyl, n-, i-, s- oder t-Butyl, n- oder i-Pentyl, n- oder i-Hexyl, n- oder i- Dodecyl und/oder oder Benzyl substituiertes Ammoniumion steht;

außerdem für Methyl, Ethyl, n- oder i-Propyl, n-, i-, s- oder t-Butyl, n- oder i-Pentyl, n- oder i-Hexyl, Fluormethyl, Trifluormethyl, Hydroxyethyl, Cyanoethyl, Methoxyethyl, Methylthioethyl, Methylaminoethyl, Ethylaminoethyl, N,N-Dimethylaminoethyl, N,N-Diethylaminoethyl, Methylcarbonylmethyl, Methylcarbonylethyl, N-Methylaminocarbonylmethyl, N-Methylaminocarbonylethyl, N-Ethylaminocarbonylmethyl, N-Ethylaminocarbonylethyl, N,N-Dimethylaminocarbonylmethyl, N,N-Diethylaminocarbonylmethyl, N,N-Dimethylaminocarbonylethyl, N,N-Diethylaminocarbonylethyl, N-Acetylaminomethyl, N-Acetylaminoethyl, N-Propionylaminomethyl, N-Propionylaminoethyl, Methylsulfonylmethyl, Methylsulfonylethyl, Ethylsulfonylmethyl, Ethylsulfonylethyl, Allyl, Propargyl, Cyclopentyl, Cyclohexyl odr für jeweils gegebenenfalls ein- bis dreifach, gleich oder verschieden substituiertes Phenyl, Pyridyl, Benzyl oder Furanylmethyl steht, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio,

$R^4$ für Methyl steht,

$R^5$ für Isopropyl steht und

X für Sauerstoff steht.

Im einzelnen seien die bei den Herstellungsbeispielen genannten Verbindungen der allgemeinen Formel (I) genannt.

Verwendet man beispielsweise 2-Methylpyrimidin-4,5-dicarbonsäurediethylester und 2-Amino-2,3-dimethylbuttersäureamid als Ausgangsverbindungen, so läßt sich der Reaktionsablauf des erfindungsgemäßen Verfahrens (a) durch das folgende Formelschema darstellen:

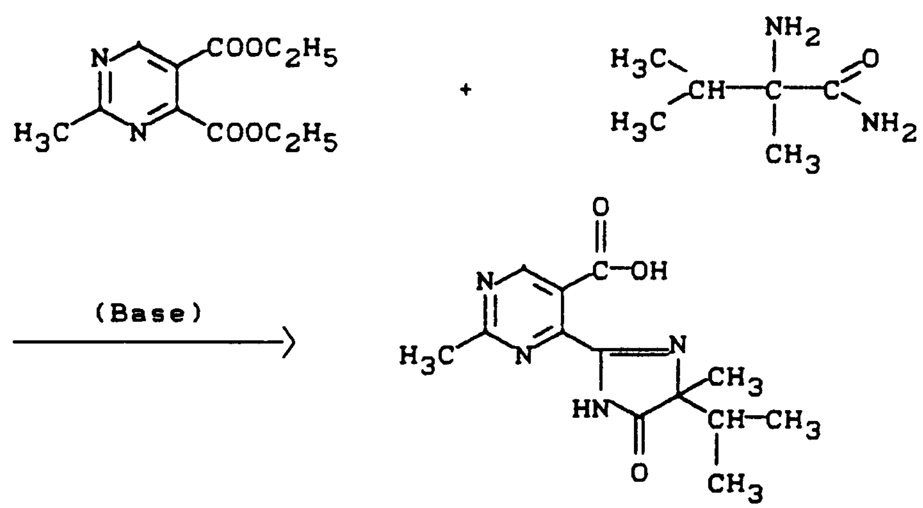


Verwendet man beispielsweise 2-Methyl-4-(4-methyl-4-isopropylimidazolin-5-on-2-yl)-pyrimidin-5-carbonsäure und Ethanol als Ausgangsstoffe sowie N,N'-Dicyclohexylcarbodiimid als Kondensationsmittel, so läßt sich der Reaktionsablauf des erfindungsgemäßen Verfahrens (b) durch das folgende Formelschema darstellen:

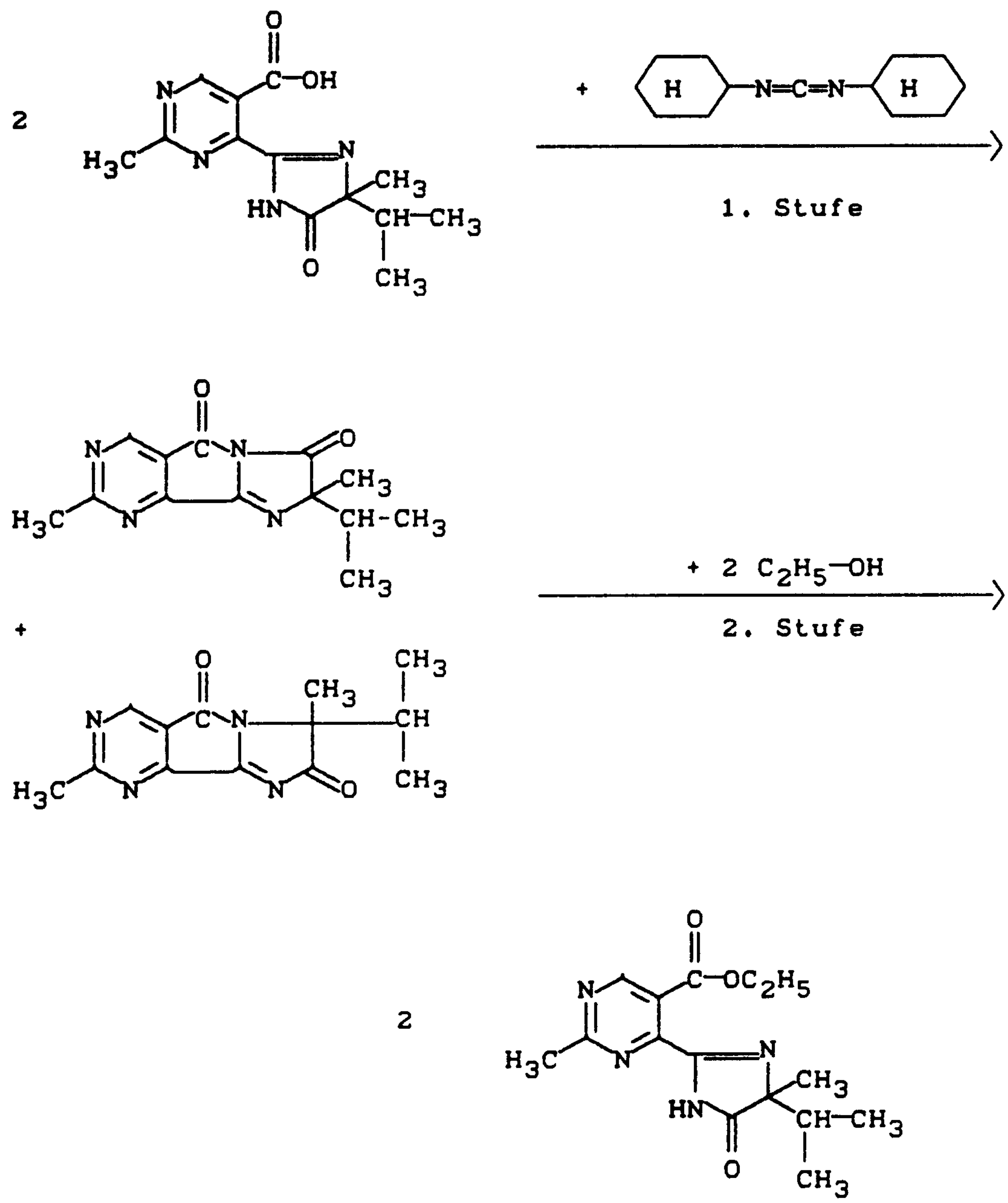


Die zur Durchführung des erfindungsgemäßen Verfahrens (a) als Ausgangsstoffe benötigten Pyrimidin-4,5-dicarbonsäurediester sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäßen Verbindungen der Formel (I) als bevorzugt für diese Substituenten genannt wurden. $R^6$ und $R^7$ stehen vorzugsweise unabhängig voneinander jeweils für geradkettiges oder verzweigtes Alkyl mit 1 bis 4 Kohlenstoffatomen, insbesondere für Methyl oder Ethyl.

Die Pyrimidin-4,5-dicarbonsäurediester der Formel (II) sind bekannt oder erhältlich in Analogie zu bekannten Verfahren (vergleiche z. B. EP 305 184; J. Chem. Soc., Perkin Trans. 1, 1980, 1667-1670; Justus Liebigs Ann. Chem. 1977, 1413-1420; J. Heterocycl. Chem. 14, 695-696 [1977]; Bull. Soc. Chim. Fr. 9-10 Pt.2,1543-1548, [1976]; Justus Liebigs Ann. Chem. 1976, 1809-1819; Chem. Ber. 108, 3877-3882 [1975]; Arch. Pharm. 308, 118-121 [1975]; JP 49024077; Justus Liebigs Ann. Chem. 1974, 1190-1194; DE 22 42 162; Chem. Pharm. Bull. 20, 1513-1521 [1972]; DE 20 46 577; J. Amer. Chem. Soc. 84, 837-844 [1962]; J. Heterocycl. Chem. 2, 202-204 [1965]; Chem. Pharm. Bull. 8, 262-264 [1960]; J. Org. Chem. 20, 1342-1346 [1955]; US 2.774.760).

Die zur Durchführung des erfindungsgemäßen Verfahrens (a) weiterhin als Edukte erforderlichen Aminosäureamide sind durch die Formel (III) allgemein definiert. In dieser Formel (III) stehen $R^4$ und $R^5$

vorzugsweise für diejenigen Reste, die bereits im Zusammenhang mit der Beschreibung der erfindungsgemäßen Stoffe der Formel (I) als bevorzugt für diese Substituenten genannt wurden. Die Aminosäureamide der Formel (III) sind allgemein bekannt oder erhältlich in Analogie zu allgemein bekannten Verfahren (vergleiche z. B. Houben-Weyl-Müller "Methoden der organischen Chemie", Thieme Verlag Stuttgart 1974; Band XV/1, S.46ff; Band XV/2, S.112ff).

Die zur Durchführung des erfindungsgemäßen Verfahrens (b) als Edukte erforderlichen Imidazolinylpyrimidincarbonsäuren sind durch die Formel (Ia) allgemein definiert. In dieser Formel (Ia) stehen $R^1$, $R^2$, $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.
Die Imidazolinylpyrimidincarbonsäuren der Formel (Ia) sind erfindungsgemäße Verbindungen und erhältlich mit Hilfe des erfindungsgemäßen Verfahrens (a).

Die zur Durchführung des erfindungsgemäßen Verfahrens (b) weiterhin als Edukte erforderlichen Alkohole und Thiole sind durch die Formel (V) allgemein definiert. In dieser Formel (V) stehen $R^3$ und X vorzugsweise für diejenigen Reste, die bereits im Zusammenhang mit der Beschreibung der erfindungsgemäßen Stoffe der Formel (I) als bevorzugt für diese Substituenten genannt wurden. Die Alkohole und Thiole der Formel (V) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (a) wird vorzugsweise in Gegenwart eines geeigneten Reaktionshilfsmittels durchgeführt. Als solche kommen alle üblichen anorganischen und organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 180 °C, vorzugsweise bei Temperaturen zwischen 20 °C und 120 °C.

Das erfindungsgemäße Verfahren (a) wird üblicherweise unter Normaldruck durchgeführt. Es ist jedoch auch möglich unter erhöhtem oder vermindertem Druck zu arbeiten,

Zur Durchführung des erfindungsgemäßen Verfahrens (a) setzt man pro Mol an Pyrimidin-4,5-dicarbonsäurediester der Formel (II) im allgemeinen 1.0 bis 1.5 Mol, vorzugsweise äquimolare Mengen an Aminosäureamid der Formel (III) und 1.0 bis 5.0 Mol, vorzugsweise 1.0 bis 3.0 Mol Base als Reaktionshilfsmittel ein.
Die Reaktionsdurchführung, Aufarbeitung und Isolierung der Reaktionsprodukte erfolgt nach bekannten Verfahren (vergleiche hierzu beispielsweise EP 41 623 oder die Herstellungsbeispiele).

Die 1. Stufe des erfindungsgemäßen Verfahrens (b) wird in Gegenwart eines geeigneten Kondensationsmittels durchgeführt. Als solche kommen alle für derartige Cyclisierungsreaktionen üblichen Kondensationsmittel infrage. Beispielhaft genannt seien Säurehalogenidbildner, wie Phosphortribromid, Phosphorpentachlorid, Phosphoroxychlorid oder Thionylchlorid, Anhydridbildner, wie Chlorameisensäureethylester oder Methansulfonylchlorid, Carbodiimide, wie N,N'-Dicyclohexylcarbodiimid (DCC) oder andere übliche Kondensationsmittel, wie N,N'-Carbonyldiimidazol, Triphenylphosphin/Tetrachlorkohlenstoff oder 2-Ethoxy-N-ethoxycarbonyl-1,2-dihydrochinolin (EEDQ).

Als Verdünnungsmittel zur Durchführung der 1. Stufe des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Alkohole, wie Methanol, Ethanol oder Isopropanol.

Die 1. Stufe des erfindungsgemäßen Verfahrens (b) kann gegebenenfalls in Gegenwart eines geeigneten Reaktionshilfsmittels durchgeführt werden. Als solche kommen alle üblichen anorganischen und organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-t-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 1. Stufe des erfindungsgemäßen Verfahrens (b) in einem größeren Bereich variiert werden. Im allgemeinen arbeitet man bei Temperaturen zwischen -20 °C und 100 °C, vorzugsweise bei Temperaturen zwischen 0 °C und 80 °C.

Zur Durchführung der 1. Stufe des erfindungsgemäßen Verfahrens (b) setzt man pro Mol Imidazolinylpyrimidin der Formel (Ia) im allgemeinen 1,0 bis 5,0 Mol, vorzugsweise 1,0 bis 1,5 Mol an Kondensationsmittel und gegebenenfalls 0,001 bis 2,0 Mol, vorzugsweise 0,1 bis 1,2 Mol Base als Reaktionshilfsmittel ein. Die Reaktionsdurchführung, Aufarbeitung und Isolierung der Reaktionsprodukte erfolgt nach bekannten Verfahren (vergleiche hierzu beispielsweise EP 41 623 oder die Herstellungsbeispiele).

Als Verdünnungsmittel zur Durchführung der 2. Stufe des erfindungsgemäßen Verfahrens (b) kommen ebenfalls inerte organische Lösungsmittel infrage. Vorzugsweise verwendet man die bei der 1. Stufe des erfindungsgemäßen Verfahrens (b) genannten Verdünnungsmittel. Es ist jedoch auch möglich, bei Verwendung von flüssigen Alkoholen oder Thiolen der Formel (V) als Reaktionskomponente, diese in einem entsprechenden Überschuß gleichzeitig als Verdünnungsmittel einzusetzen.

Die 2. Stufe des erfindungsgemäßen Verfahrens (b) kann gegebenenfalls in Gegenwart eines geeigneten basischen Reaktionshilfsmittels durchgeführt werden. Als solche kommen alle üblichen anorganischen oder organischen Basen in Frage. Hierzu gehören beispielsweise Erdalkali- oder Alkalimetallhydroxide wie Natriumhydroxid, Calciumhydroxid, Kaliumhydroxid oder auch Ammoniumhydroxid, Alkalimetallcarbonate wie Natriumcarbonat, Kaliumcarbonat, Kaliumhydrogencarbonat, Natriumhydrogencarbonat oder Ammoniumcarbonat, Alkali- oder Erdalkalimetallacetate wie Natriumacetat, Kaliumacetat, Calciumacetat oder Ammoniumacetat sowie tertiäre Amine wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 2. Stufe des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 20 °C und 100 °C.

Die 2. Stufe des erfindungsgemäßen Verfahrens (b) wird üblicherweise unter Normaldruck durchgeführt.

Zur Durchführung der 2. Stufe des erfindungsgemäßen Verfahrens (b) setzt man pro Mol Imidazopyrrolo-pyrimidin der Formel (IVa) bzw, (IVb) im allgemeinen 1.0 bis 50 Mol, vorzugsweise 1,0 bis 10 Mol Alkohol oder Thiol der Formel (V) und gegebenenfalls 0.1 bis 2.0 Mol, vorzugsweise 0,5 bis 1.2 Mol Base als Reaktionshilfsmittel ein.

Die Reaktionsdurchführung, Aufarbeitung und Isolierung der Reaktionsprodukte erfolgt nach bekannten Verfahren (vergleiche hierzu beispielsweise EP 41 623 oder die Herstellungsbeispiele).

In einer besonderen Durchführungsform ist es auch möglich, die 1. und die 2. Stufe des erfindungsgemäßen Verfahrens (b) in einem Reaktionsschritt in einem sogenannten "Eintopfverfahren" durchzuführen. Man geht dabei von Imidazolinylpyrimidinen der Formel (Ia) aus und setzt diese nacheinander im "Eintopfverfahren" zunächst mit einem Kondensationsmittel und anschließend mit einem Alkohol oder Thiol der Formel (V) um.

Die Reinigung der Endprodukte der Formel (I) erfolgt mit Hilfe üblicher Verfahren, beispielsweise durch Säulenchromatographie oder durch Umkristallisieren.

Erfindungsgemäße Verbindungen der Formel (I), bei welchen $R^3$ für Wasserstoff steht, können mit Hilfe üblicher Methoden in Salze überführt werden, beispielsweise indem man sie in einem geeigneten inerten Lösungsmittel löst, anschließend eine entsprechende Base zugibt und das Salz durch Abfiltrieren oder Abdestillieren des Lösungsmittels isoliert und gegebenenfalls durch Waschen mit einem inerten Lösungsmittel oder durch Umkristallisieren reinigt.

Die Charakterisierung der Verbindung der Formel (I) erfolgt mit Hilfe des Schmelzpunktes oder bei nicht kristallisierenden Verbindungen mit Hilfe der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle

Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern in monokotylen und dikotylen Kulturen wie beispielsweise Weizen oder Soja einsetzen.

Daneben zeigen die erfindungsgemäßen Wirkstoffe in entsprechenden Aufwandmengen auch blattinsektizide und fungizide Wirksamkeiten und lassen sich beispielsweise zur Bekämpfung von Reiskrankheiten, wie beispielsweise gegen den Erreger der Reisfleckenkrankheit (Pyricularia oryzae) einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden.

Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol,

Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitro-benzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyl-iden]-cyclohexancarbonsäure (ALLOXYDIM); 4-Amino-benzolsulfonyl-methylcarbamat (ASULAM); 2-Chlor-4-ethylamino-6-isopropylamino-1,3,5-triazin (ATRAZIN); 2-[[[[(4,6-Dimethoxypyrimidin-2-yl)-amino)-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); 5-Amino-4-chlor-2-phenyl-2,3-dihydro-3-oxypyridazin (CHLORIDAZON); Ethyl-2-{[(4-chlor-6-methoxy-2-pyrimidinyl)-aminocarbonyl)-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)harnstoff (CHLORTOLURON); exo-1-Methyl-4-(1-methylethyl)-2-(2-methylphenyl-methoxy)-7-oxabicyclo-(2,2,1)heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexylthiolcarbamat (CYCLOATE); 2-[1-(Ethoximino)-butyl]-3-hydroxy-5-[tetrahydro-(2H)-thiopyran-3-yl)-2-cyclohexen-1-on (CYCLOXYDIM); 2-[4-(2,4-Dichlorphenoxy)-phenoxy]propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethyl-thio-1,2,4-triazin-5(4H)-on (ETHIOZIN); 2-{4-[(6-Chlor-2-benzoxazolyl)-oxy]-phenoxy}-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxyl-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-(4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl)-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Me-thyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylenthiolcarbamat (MOLINATE); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitrophenyl)ether (OXYFLUORFEN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); $\alpha$-Chlor-2',6'-diethyl-N-(2-propoxyethyl)-acetanilid (PRETILACHLOR); 2-Chlor-N-isopropylacetanilid (PROPACHLOR); Isopropyl-N-phenylcarbamat (PROPHAM); 0-(6-Chlor-3-phenyl-pyridazin-4-yl)-S-octyl-thiocarbonat (PYRIDATE); 2-[4-(6-Chlor-chinoxalin-2-yl-oxy)-phenoxy]-propionsäureethylester (QUIZALOFOPETHYL); 2-[1-(Ethoxamino)-butyl-iden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl)-N,N-diethyl-thiocarbamat (THIOBENCARB); N,N-Diisopropyl-S-(2,3,3-trichlorallyl)-thiolcarbamat (TRIALLATE) oder 2,6-Dinitro-4-trifluormethyl-N,N-dipropylanilin (TRIFLURALIN) sind gegebenenfalls von Vorteil. Einige Mischungen zeigen überraschenderweise auch synergistische Wirkungen.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro Hektar.

Die Herstellung und die Verwendung der erfindungsgemäßen Wirkstoffe geht aus den nachfolgenden Beispielen hervor.

Herstellungsbeispiele

Beispiel 1

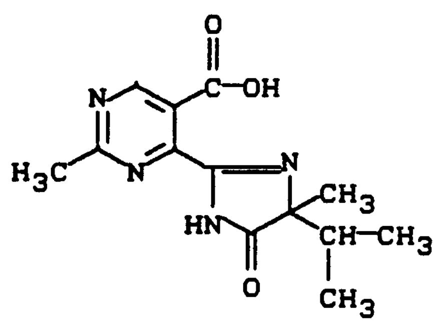

(Verfahren a)

Zu einer Mischung von 98,8 g (0,415 Mol) 2-Methylpyrimidin-4,5-dicarbonsäurediethylester (vergleiche z. B. J. Heterocycl. Chem. 2, 202-204 [1965]) und 54,0 g (0,415 Mol) 2-Amino-2,3-dimethylbuttersäureamid in 700 ml wasserfreiem Toluol gibt man portionsweise 102,2 g (0,913 Mol) Kalium-tert.-butylat und rührt anschließend 16 Stunden bei 80 °C. Zur Aufarbeitung wird die Reaktionsmischung abgekühlt, der ausgefallene Feststoff filtriert, mehrfach mit Diethylether nachgewaschen und anschließend in Wasser gelöst. Die wässrige Lösung wird mit halbkonzentrierter Salzsäure auf pH 5 gebracht und fünfmal mit jeweils 200 ml Dichlormethan extrahiert. Die vereinigten organischen Extrakte werden über Natriumsulfat getrocknet und im Vakuum eingeengt. Der Rückstand läßt sich durch Chromatographie an Kieselgel reinigen.

Man erhält 59 g (51 % der Theorie) 2-Methyl-4-(4-methyl-4-isopropyl-imidazolin-5-on-2-yl)-pyrimidin-5-carbonsäure vom Schmelzpunkt 56-57 °C.

Beispiel 2

1. Stufe (IV-1)

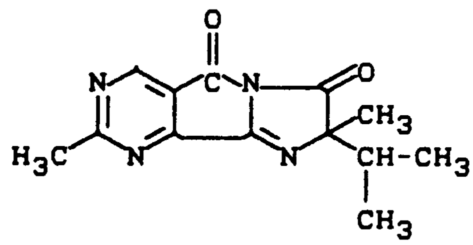

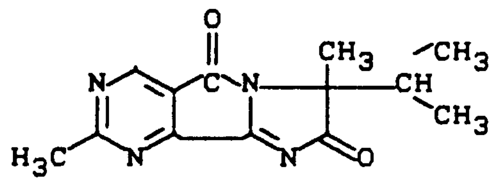

22,8 g (0,082 Mol) 2-Methyl-4-(4-methyl-4-isopropyl-imidazolin-5-on-2-yl)-pyrimidin-5-carbonsäure werden in 150 ml Tetrahydrofuran suspendiert, mit 17,0 g (0,082 Mol) N,N'-Dicyclohexylcarbodiimid versetzt, eine Stunde bei 40 ° C gerührt, anschließend im Vakuum eingeengt und der Rückstand chromatographisch an Kieselgel gereinigt (Laufmittel: Essigsäureethylester/Cyclohexan 1:1).

Man erhält 16,4 g (77 % der Theorie) einer Isomerenmischung des oben angegebenen Imidazo-pyrrolo-pyrimidins als Öl.

$^1$H-NMR ($CDCl_3$/Tetramethylsilan):
$\delta$ = 0,95 (d,3H); 1,15 (d,3H); 1,58 (s,3H); 1,9 (m,1H); 3,02 (s,3H); 9,32 (s,1H) ppm.

2. Stufe

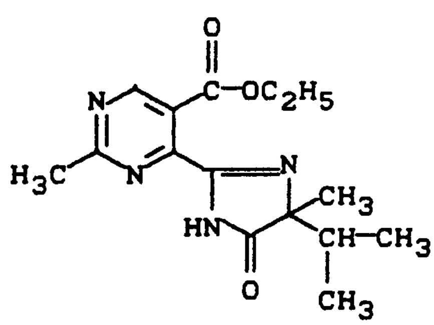

4,65 g (0,018 Mol) der aus der 1. Stufe erhaltenen Imidazo-pyrrolo-pyrimidin-Mischung werden mit 20 ml Ethanol versetzt und 18 Stunden bei Raumtemperatur gerührt. Zur Aufarbeitung wird überschüssiges Ethanol im Vakuum abdestilliert, der Rückstand in Essigsäureethylester aufgenommen, mehrmals mit Wasser gewaschen, über Natriumsulfat getrocknet, im Vakuum eingeengt und der Rückstand durch Chromatographie an Kieselgel (Laufmittel: Essigsäureethylester/n-Hexan 3:1) gereinigt.

Man erhält 3,56 g (65 % der Theorie) 2-Methyl-4-(4-methyl-4-isopropyl-imidazolin-5-on-2-yl)-pyrimidin-5-carbonsäureethylester als Öl.

$^1$H-NMR ($CDCl_3$/Tetramethylsilan):
$\delta$ = 0,87 (d,3H); 1,05 (d,3H); 1,37 (t,3H); 1,38 (s,3H); 2,09 (m,1H); 4,40 (m/2H); 8,86 (s,1H); 9,11 (s,1H) ppm.

In entsprechender Weise und gemäß den allgemeinen Angaben zur Herstellung erhält man die folgenden Imidazolinylpyrimidine der allgemeinen Formel (I):

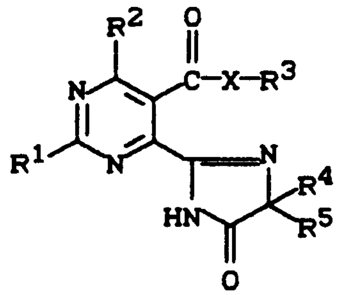

(I)

| Bsp.Nr. | $R^1$ | $R^2$ | $R^3$ | $R^4$ | $R^5$ | X | physikalische Eigenschaften |
|---|---|---|---|---|---|---|---|
| 3 | $i\text{-}C_3H_7$ | H | H | $CH_3$ | $i\text{-}C_3H_7$ | O | Fp. 98-99° C |
| 4 | $H_3C\text{-}S\text{-}$ | H | H | $CH_3$ | $i\text{-}C_3H_7$ | O | Fp. 121-123° C |
| 5 | $t\text{-}C_4H_9$ | H | H | $CH_3$ | $i\text{-}C_3H_7$ | O | Fp. 170-171° C |
| 6 | $H_5C_2\text{-}S\text{-}$ | H | H | $CH_3$ | $i\text{-}C_3H_7$ | O | Fp. 108-109° C |
| 7 | $H_5C_2\text{-}O\text{-}$ | H | H | $CH_3$ | $i\text{-}C_3H_7$ | O | Fp. 158-159° C |
| 8 | $(CH_3)_2N\text{-}$ | H | H | $CH_3$ | $i\text{-}C_3H_7$ | O | Fp. 178-179° C |
| 9 | $C_6H_5$ | H | H | $CH_3$ | $i\text{-}C_3H_7$ | O | Fp. 205-206° C |
| 10 | H | H | H | $CH_3$ | $i\text{-}C_3H_7$ | O | Fp. 210-211° C |
| 11 | $H_3C\text{-}NH\text{-}$ | H | H | $CH_3$ | $i\text{-}C_3H_7$ | O | Fp. 198-199° C |
| 12 | $H_5C_6\text{-}CH_2\text{-}S\text{-}$ | H | H | $CH_3$ | $i\text{-}C_3H_7$ | O | Fp. 187-188° C |
| 13 | $H_3C\text{-}O\text{-}$ | H | H | $CH_3$ | $i\text{-}C_3H_7$ | O | Fp. 140-141° C |
| 14 | $H_3C\text{-}S\text{-}CH_2\text{-}$ | H | H | $CH_3$ | $i\text{-}C_3H_7$ | O | Fp. 98-99° C |

| Bsp.Nr. | $R^1$ | $R^2$ | $R^3$ | $R^4$ | $R^5$ | X | physikalische Eigenschaften |
|---|---|---|---|---|---|---|---|
| 15 | $CH_3$ | H | $K^{\oplus}$ | $CH_3$ | $i\text{-}C_3H_7$ | O | Fp. 248-249° C |
| 16 | $H_3C\text{-}NH\text{-}$ | H | $K^{\oplus}$ | $CH_3$ | $i\text{-}C_3H_7$ | O | Fp. 238-239° C |
| 17 | $H_2N\text{-}$ | H | H | $CH_3$ | $i\text{-}C_3H_7$ | O | Fp. 191-192° C |
| 18 | $n\text{-}C_4H_9$ | H | H | $CH_3$ | $i\text{-}C_3H_7$ | O | Fp. 102-103° C |
| 19 | $n\text{-}C_3H_7$ | H | H | $CH_3$ | $i\text{-}C_3H_7$ | O | Fp. 102-103° C |
| 20 | $C_2H_5$ | H | H | $CH_3$ | $i\text{-}C_3H_7$ | O | Fp. 127-128° C |
| 21 | $CH_3$ | H | $CH_3$ | $CH_3$ | $i\text{-}C_3H_7$ | O | $^1$H-NMR*): 0,87; 1,06; 1,38; 2,09; 2,82; 3,91; 8,71; 8,85 |
| 22 | 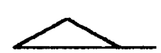 | H | H | $CH_3$ | $i\text{-}C_3H_7$ | O | Fp. 140-141° C |

*) Die $^1$H-NMR-Spektren wurden in Deuterochloroform ($CDCl_3$) oder Hexadeuterodimethylsulfoxid (DMSO-$d_6$) mit Tetramethylsilan (TMS) als innerem Standard aufgenommen. Angegeben ist die chemische Verschiebung als δ-Wert in ppm.

| Bsp.Nr. | $R^1$ | $R^2$ | $R^3$ | $R^4$ | $R^5$ | X | physikalische Eigenschaften |
|---|---|---|---|---|---|---|---|
| 23 | $CH_3$-O-$CH_2$- / $C_2H_5$-O-$CH_2$- (2:1) | H | H | $CH_3$ | i-$C_3H_7$ | O | $^1$H-NMR*): 0,81 (d); 0,98 (d); 1,17 (t); 1,26 (s); 1,95 (m); 3,42 (s); 3,62 (q); 4,72 (s); 9,25 (s) |
| 24 | $CH_3$ | H | -$CH_2$-C($CH_3$)$_3$ | $CH_3$ | i-$C_3H_7$ | O | Fp. 84-85°C |
| 25 | $CH_3$-O-C(O)-NH- | H | H | $CH_3$ | i-$C_3H_7$ | O | $^1$H-NMR*): 0,83 (d); 0,97 (d); 1,2 (s); 1,91 (m); 3,7 (s); 4,15 (s); 9,12 (s); 11,09 (s) |
| 26 | $CH_3$ | H | -$CH_2$-$C_6H_5$ | $CH_3$ | i-$C_3H_7$ | O | Fp. 72°C |
| 27 | $CH_3$ | H | -$CH_2$-C≡CH | $CH_3$ | i-$C_3H_7$ | O | Fp. 76-77°C |
| 28 | $CH_3$ | H | 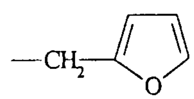 | $CH_3$ | i-$C_3H_7$ | O | Fp. 44-45°C |
| 29 | $C_2H_5$-O-$CH_2$- | H | H | $CH_3$ | i-$C_3H_7$ | O | $^1$H-NMR*): 0,92 (d); 1,14 (d); 1,35 (t); 1,54 (s); 3,76 (q); 4,89 (s); 9,93 (s) |

| Bsp.Nr. | $R^1$ | $R^2$ | $R^3$ | $R^4$ | $R^5$ | X | physikalische Eigenschaften |
|---|---|---|---|---|---|---|---|
| 30 | $CH_3$ | H | $Na^+$ | $CH_3$ | i-$C_3H_7$ | O | Fp. >260°C |
| 31 | $CF_3$ | H | H | $CH_3$ | i-$C_3H_7$ | O | Fp. 164-165°C |
| 32 | $CH_3$ | H | $NH_4^+$ | $CH_3$ | i-$C_3H_7$ | O | Fp. >260°C |
| 33 | $CH_3$ | H | 1/2 $Ca^{2+}$ | $CH_3$ | i-$C_3H_7$ | O | Fp. >260°C |
| 34 | H | H | $C_2H_5$ | $CH_3$ | i-$C_3H_7$ | O | Fp. 58-59°C |
| 35 | $CH_3$ | H | n-$C_3H_7$ | $CH_3$ | i-$C_3H_7$ | O | Fp. 84-85°C |
| 36 | 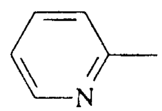 | H | H | $CH_3$ | i-$C_3H_7$ | O | Fp. 206-207°C |
| 37 | 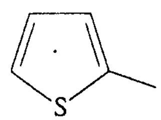 | H | H | $CH_3$ | i-$C_3H_7$ | O | Fp. 212-213°C |
| 38 | 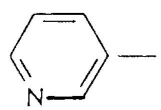 | H | H | $CH_3$ | i-$C_3H_7$ | O | Fp. 242-243°C |
| 39 | $CH_3$ | H | i-$C_3H_7$ | $CH_3$ | i-$C_3H_7$ | O | Fp. 98-99°C |

| Bsp.Nr. | $R^1$ | $R^2$ | $R^3$ | $R^4$ | $R^5$ | X | physikalische Eigenschaften |
|---|---|---|---|---|---|---|---|
| 40 | $CH_3$ | H | $-CH_2-CH=CH_2$ | $CH_3$ | $i-C_3H_7$ | O | Fp. 78-79°C |
| 41 | $CH_3$ | H | $n-C_4H_9$ | $CH_3$ | $i-C_3H_7$ | O | Fp. 88-89°C |
| 42 | 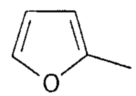 | H | H | $CH_3$ | $i-C_3H_7$ | O | Fp. 239-240°C |
| 43 | $C_6H_5-CH_2-$ | H | H | $CH_3$ | $i-C_3H_7$ | O | Fp. 147°C |
| 44 | H | H | $CH_3$ | $CH_3$ | $i-C_3H_7$ | O | Fp. 92-93°C |
| 45 | H | H | $-CH_2-C_6H_5$ | $CH_3$ | $i-C_3H_7$ | O | $^1H$-NMR*): 0,86 (d); 1,03 (d); 1,36 (s); 2,07 (m); 5,38 (m); 7,38 (m); 8,74 (s); 8,94 (s); 9,33 (s) |
| 46 | H | H | $n-C_4H_9$ | $CH_3$ | $i-C_3H_7$ | O | Fp. 63-64°C |
| 47 | H | H | $-CH_2-CH=CH_2$ | $CH_3$ | $i-C_3H_7$ | O | Fp. 124-125°C |
| 48 | H | H | $-CH_2-C\equiv CH$ | $CH_3$ | $i-C_3H_7$ | O | $^1H$-NMR*): 0,87 (d); 1,06 (d); 1,4 (s); 2,11 (m); 2,56 (t); 4,93 (d); 8,94 (s); 8,97 (s); 9,37 (s) |

| Bsp.Nr. | $R^1$ | $R^2$ | $R^3$ | $R^4$ | $R^5$ | X | physikalische Eigenschaften |
|---|---|---|---|---|---|---|---|
| 49 | H | H | n-$C_3H_7$ | $CH_3$ | i-$C_3H_7$ | O | Fp. 90-91°C |
| 50 | H | H | $-CH_2-C(CH_3)_3$ | $CH_3$ | i-$C_3H_7$ | O | Fp. 98-99°C |
| 51 | $C_2H_5$ | H | $CH_3$ | $CH_3$ | i-$C_3H_7$ | O | Fp. 124-125°C |
| 52 | $C_2H_5$ | H | $C_2H_5$ | $CH_3$ | i-$C_3H_7$ | O | Fp. 105-106°C |
| 53 | $C_2H_5$ | H | n-$C_3H_7$ | $CH_3$ | i-$C_3H_7$ | O | Fp. 98-99°C |
| 54 | $C_2H_5$ | H | i-$C_3H_7$ | $CH_3$ | i-$C_3H_7$ | O | Fp. 137-138°C |
| 55 | $C_2H_5$ | H | n-$C_4H_9$ | $CH_3$ | i-$C_3H_7$ | O | Fp. 128-129°C |
| 56 | $C_2H_5$ | H | $-CH_2-C_6H_5$ | $CH_3$ | i-$C_3H_7$ | O | Fp. 102-103°C |
| 57 | $C_2H_5$ | H | $-CH_2-CF_3$ | $CH_3$ | i-$C_3H_7$ | O | Fp. 145-146°C |
| 58 | $C_2H_5$ | H | $-CH_2-CH=CH_2$ | $CH_3$ | i-$C_3H_7$ | O | Fp. 109-110°C |
| 59 | $C_2H_5$ | H | $-CH_2-C\equiv CH$ | $CH_3$ | i-$C_3H_7$ | O | Fp. 138-139°C |

| Bsp.Nr. | $R^1$ | $R^2$ | $R^3$ | $R^4$ | $R^5$ | X | physikalische Eigenschaften |
|---|---|---|---|---|---|---|---|
| 60 | $C_2H_5$ | H | $s-C_4H_9$ | $CH_3$ | $i-C_3H_7$ | O | Fp. 98-99°C |
| 61 | $C_2H_5$ | H | 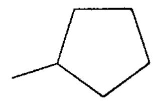 | $CH_3$ | $i-C_3H_7$ | O | Fp. 148-149°C |
| 62 | $NH_2$ | H | $n-C_3H_7$ | $CH_3$ | $i-C_3H_7$ | O | Fp. 130-131°C |
| 63 | $NH_2$ | H | $-CH_2-C_6H_5$ | $CH_3$ | $i-C_3H_7$ | O | $^1H$-NMR*): 0,88 (d); 1,02 (d); 1,33 (s); 2,06 (m); 5,31 (m); 5,49 (s); 7,36 (m); 8,55 (s); 8,68 (s) |
| 64 | $C_2H_5$ | H | 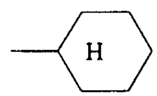 | $CH_3$ | $i-C_3H_7$ | O | Fp. 84-85°C |
| 65 | $C_2H_5$ | H | $-CH_2-CH_2-S-CH_3$ | $CH_3$ | $i-C_3H_7$ | O | Fp. 93-94°C |
| 66 | $C_2H_5$ | H | $-CH_2-CH_2-NH-CH_3$ | $CH_3$ | $i-C_3H_7$ | O | Fp. 91-92°C |
| 67 | $C_2H_5$ | H | $-CH_2-C(O)-CH_3$ | $CH_3$ | $i-C_3H_7$ | O | Fp. 93-94°C |
| 68 | $n-C_3H_7$ | H | $CH_3$ | $CH_3$ | $i-C_3H_7$ | O | Fp. 43-44°C |
| 69 | $n-C_3H_7$ | H | $C_2H_5$ | $CH_3$ | $i-C_3H_7$ | O | Fp. 81-82°C |

| Bsp.Nr. | $R^1$ | $R^2$ | $R^3$ | $R^4$ | $R^5$ | X | physikalische Eigenschaften |
|---|---|---|---|---|---|---|---|
| 70 | $n-C_3H_7$ | H | $n-C_3H_7$ | $CH_3$ | $i-C_3H_7$ | O | Fp. 84-85°C |
| 71 | $n-C_3H_7$ | H | $-CH_2-C\equiv CH$ | $CH_3$ | $i-C_3H_7$ | O | Fp. 88-89°C |
| 72 | $CH_3$ | H | $-CH_2-CF_3$ | $CH_3$ | $i-C_3H_7$ | O | Fp. 128-129°C |
| 73 | $CH_3$ | H | $-CH_2-C(O)-CH_3$ | $CH_3$ | $i-C_3H_7$ | O | $^1$H-NMR*): 0,85 (d); 1,03 (d); 1,36 (s); 2,07 (m); 2,25 (s); 2,83 (s); 4,88 (m); 8,84 (s); 8,99 (s) |
| 74 | $C_2H_5-S-$ | H | $CH_3$ | $CH_3$ | $i-C_3H_7$ | O | Fp. 88-89°C |
| 75 | $C_2H_5-S-$ | H | $C_2H_5$ | $CH_3$ | $i-C_3H_7$ | O | Fp. 104-105°C |
| 76 | $C_2H_5$ | H | $-CH_2-CH_2-NH_2$ | $CH_3$ | $i-C_3H_7$ | O | Fp. 135-136°C |
| 77 | $C_2H_5$ | H | 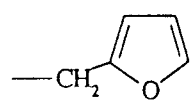 | $CH_3$ | $i-C_3H_7$ | O | Fp. 109-110°C |
| 78 | 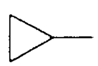 | H | $CH_3$ | $CH_3$ | $i-C_3H_7$ | O | Fp. 130-131°C |
| 79 | 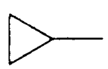 | H | $n-C_3H_7$ | $CH_3$ | $i-C_3H_7$ | O | Fp. 110-111°C |

| Bsp.Nr. | $R^1$ | $R^2$ | $R^3$ | $R^4$ | $R^5$ | X | physikalische Eigenschaften |
|---|---|---|---|---|---|---|---|
| 80 | 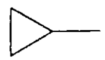 | H | i-$C_3H_7$ | $CH_3$ | i-$C_3H_7$ | O | Fp. 126-127°C |
| 81 | 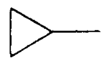 | H | -$CH_2$-CH=$CH_2$ | $CH_3$ | i-$C_3H_7$ | O | Fp. 128-129°C |
| 82 | n-$C_4H_9$ | H | $CH_3$ | $CH_3$ | i-$C_3H_7$ | O | $^1$H-NMR*): 0,87 (d); 0,97 (t); 1,06 (d); 1,42 (m); 1,83 (m); 2,09 (m); 3,04 (m); 3,92 (s); 8,74 (s); 8,85 (s) |
| 83 | n-$C_4H_9$ | H | $C_2H_5$ | $CH_3$ | i-$C_3H_7$ | O | Fp. 68-69°C |
| 84 | n-$C_4H_9$ | H | n-$C_3H_7$ | $CH_3$ | i-$C_3H_7$ | O | Fp. 70-71°C |
| 85 | i-$C_3H_7$ | H | $CH_3$ | $CH_3$ | i-$C_3H_7$ | O | Fp. 84-85°C |
| 86 | i-$C_3H_7$ | H | $C_2H_5$ | $CH_3$ | i-$C_3H_7$ | O | Fp. 115-116°C |
| 87 | i-$C_3H_7$ | H | -$CH_2$-CH=$CH_2$ | $CH_3$ | i-$C_3H_7$ | O | Fp. 120-121°C |
| 88 | $CH_3$-O- | H | $CH_3$ | $CH_3$ | i-$C_3H_7$ | O | Fp. 132-133°C |

| Bsp.Nr. | $R^1$ | $R^2$ | $R^3$ | $R^4$ | $R^5$ | X | physikalische Eigenschaften |
|---|---|---|---|---|---|---|---|
| 89 | 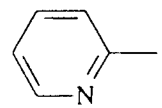 | H | $CH_3$ | $CH_3$ | i-$C_3H_7$ | O | $^1$H-NMR*): 0,88 (d); 1,08 (d); 1,39 (s); 2,1 (m); 7,51 (m); 7,95 (m); 8,62 (m); 8,8 (m); 9,07 (s) |
| 90 | 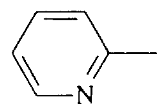 | H | $C_2H_5$ | $CH_3$ | i-$C_3H_7$ | O | Fp. 104-105°C |
| 91 | 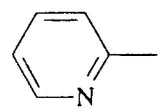 | H | $-CH_2-CH=CH_2$ | $CH_3$ | i-$C_3H_7$ | O | Fp. 78-79°C |
| 92 | 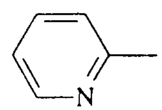 | H | $-CH_2-C\equiv CH$ | $CH_3$ | i-$C_3H_7$ | O | Fp. 156-157°C |
| 93 | 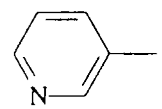 | H | n-$C_3H_7$ | $CH_3$ | i-$C_3H_7$ | O | $^1$H-NMR*): 0,88 (d); 1,01 (t); 1,07 (d); 1,4 (s); 1,75 (m); 2,11 (m); 4,3 (m); 7,4 (m); 7,78 (m); 8,9 (m); 9,03 (s); 9,09 (m) |

| Bsp.Nr. | $R^1$ | $R^2$ | $R^3$ | $R^4$ | $R^5$ | X | physikalische Eigenschaften |
|---|---|---|---|---|---|---|---|
| 94 | 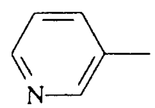 | H | $-CH_2-CH=CH_2$ | $CH_3$ | $i-C_3H_7$ | O | $^1H$-NMR*): 0,9 (d); 1,03 (d); 1,4 (s); 2,08 (m); 3,81 (m); 4,85 (m); 5,39 (m); 6,01 (m); 7,75 (m); 8,91 (m); 9,0 (m); 9,05 (m); 10,0 (m) |
| 95 | 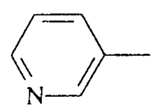 | H | $-CH_2-C\equiv CH$ | $CH_3$ | $i-C_3H_7$ | O | $^1H$-NMR*): 0,9 (d); 1,08 (d); 1,43 (s); 2,11 (m); 2,56 (m); 4,95 (m); 7,51 (m); 8,72 (m); 8,77 (m); 9,01 (s); 9,69 (m) |
| 96 | $C_2H_5$ | H | $n-C_6H_{13}$ | $CH_3$ | $i-C_3H_7$ | O | Fp. 87-88°C |
| 97 | $C_2H_5$ | H | $-CH_2-CH_2-O-CH_3$ | $CH_3$ | $i-C_3H_7$ | O | Fp. 83-84°C |
| 98 | $C_2H_5$ | H | $-CH_2-CH_2-CH(CH_3)_2$ | $CH_3$ | $i-C_3H_7$ | O | Fp. 90-91°C |
| 99 | 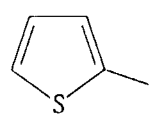 | H | $-CH_2-CH_2-CH(CH_3)_2$ | $CH_3$ | $i-C_3H_7$ | O | Fp. 131-132°C |

| Bsp.Nr. | $R^1$ | $R^2$ | $R^3$ | $R^4$ | $R^5$ | X | physikalische Eigenschaften |
|---|---|---|---|---|---|---|---|
| 100 | 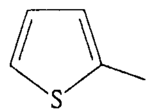 | H | $-CH_2-C\equiv CH$ | $CH_3$ | $i-C_3H_7$ | O | Fp. 158-159°C |
| 101 | 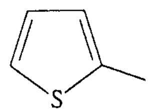 | H | $-CH_2-C(O)-CH_3$ | $CH_3$ | $i-C_3H_7$ | O | Fp. 133-134°C |
| 102 | 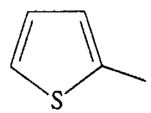 | H | $n-C_6H_{13}$ | $CH_3$ | $i-C_3H_7$ | O | Fp. 98-99°C |
| 103 | $C_2H_5-O-CH_2-$ | H | $CH_3$ | $CH_3$ | $i-C_3H_7$ | O | -- |
| 104 | $C_6H_5$ | H | $C_2H_5$ | $CH_3$ | $i-C_3H_7$ | O | Fp. 123-124°C |
| 105 | $C_6H_5$ | H | $i-C_3H_7$ | $CH_3$ | $i-C_3H_7$ | O | Fp. 155-156°C |
| 106 | $C_6H_5$ | H | $-CH_2-CH_2-CH(CH_3)_2$ | $CH_3$ | $i-C_3H_7$ | O | Fp. 168-169°C |
| 107 | $C_6H_5-CH_2-$ | H | $n-C_4H_9$ | $CH_3$ | $i-C_3H_7$ | O | $^1H$-NMR*): 0,77 (d); 0,88 (t); 0,96 (d); 1,21 (s); 1,31 (m); 1,62 (m); 1,91 (m); 4,22 (m); 4,32 (m); 7,3 (m); 9,1 (s); 11,59 (s) |

| Bsp.Nr. | $R^1$ | $R^2$ | $R^3$ | $R^4$ | $R^5$ | X | physikalische Eigenschaften |
|---|---|---|---|---|---|---|---|
| 108 | $C_6H_5-CH_2-$ | H | $-CH_2-CH=CH_2$ | $CH_3$ | $i-C_3H_7$ | O | Fp. 44-45°C |
| 109 | 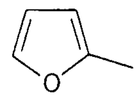 | H | $-CH_2-C(O)-CH_3$ | $CH_3$ | $i-C_3H_7$ | O | Fp. 64-65°C |
| 110 | 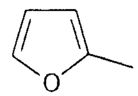 | H | $-CH_2-CF_3$ | $CH_3$ | $i-C_3H_7$ | O | Fp. 174-175°C |
| 111 | $C_6H_5-S-CH_2-$ | H | $-CH_2-CH=CH_2$ | $CH_3$ | $i-C_3H_7$ | O | $^1H$-NMR*): 0,84 (d); 1,1 (d); 1,34 (s); 2,05 (m); 4,42 (m); 4,5 (m); 4,75 (m); 5,32 (m); 5,95 (m); 7,3 (m); 8,5 (s); 8,71 (s) |
| 112 | $C_6H_5-S-CH_2-$ | H | $-CH_2-C\equiv CH$ | $CH_3$ | $i-C_3H_7$ | O | $^1H$-NMR*): 0,85 (d); 1,02 (d); 1,35 (s); 2,02 (m); 4,52 (m); 4,45 (s); 4,87 (m); 7,35 (m); 8,68 (s); 8,75 (s) |
| 113 | $C_6H_5-S-CH_2-$ | H | $-CH_2-C(O)-CH_3$ | $CH_3$ | $i-C_3H_7$ | O | Fp. 84-85°C |
| 114 | $C_6H_5-S-CH_2-$ | H | $-CH_2-C_6H_5$ | $CH_3$ | $i-C_3H_7$ | O | Fp. 78-79°C |
| 115 | $C_2H_5-O-$ | H | $CH_3$ | $CH_3$ | $i-C_3H_7$ | O | Fp. 115-116°C |

| Bsp.Nr. | $R^1$ | $R^2$ | $R^3$ | $R^4$ | $R^5$ | X | physikalische Eigenschaften |
|---|---|---|---|---|---|---|---|
| 116 | $C_6H_5$-S-$CH_2$- | H | n-$C_6H_{13}$ | $CH_3$ | i-$C_3H_7$ | O | Fp. 58-59°C |
| 117 | $C_6H_5$-S-$CH_2$- | H | -$CH_2$-$CF_3$ | $CH_3$ | i-$C_3H_7$ | O | Fp. 74-75°C |
| 118 | $C_2H_5$-O- | H | $C_2H_5$ | $CH_3$ | i-$C_3H_7$ | O | Fp. 88-89°C |
| 119 | $C_2H_5$ | H | -$CH_2$-$CH_2$-CN | $CH_3$ | i-$C_3H_7$ | O | Fp. 140-141°C |
| 120 | $C_2H_5$ | H | -CH($CH_3$)-CN | $CH_3$ | i-$C_3H_7$ | O | Fp. 130-111°C |
| 121 | $C_2H_5$ | H | -$CH_2$-CN | $CH_3$ | i-$C_3H_7$ | O | Fp. 118-119°C |

NMR

*) Die $^1$H-NMR-Spektren wurden in Deuterochloroform ($CDCl_3$) oder Hexadeuterodimethylsulfoxid (DMSO-$d_6$) mit Tetramethylsilan (TMS) als innerem Standard aufgenommen. Angegeben ist die chemische Verschiebung als δ-Wert in ppm.

**Tabelle 2:** Vorprodukte der Formel (IV)

| Bsp.Nr. | $R^1$ | $R^2$ | $R^4$ | $R^5$ | physikalische Eigenschaften |
|---|---|---|---|---|---|
| IV-2 | H | H | $CH_3$ | i-$C_3H_7$ | $^1$H-NMR*): 1,17 (m); 1,56 (s); 2,24 (m); 9,15 (s); 9,44 (s) |
| IV-3 | $C_2H_5$ | H | $CH_3$ | i-$C_3H_7$ | Fp 101-102°C |

*) Die $^1$H-NMR-Spektren wurden in Deuterochloroform ($CDCl_3$) oder Hexadeuterodimethylsulfoxid (DMSO-$d_6$) mit Tetramethylsilan (TMS) als innerem Standard aufgenommen. Angegeben ist die chemische Verschiebung als δ-Wert in ppm.

Anwendungsbeispiele:

In den folgenden Anwendungsbeispielen wurde die nachstehend aufgeführte Verbindung als Vergleichssubstanz eingesetzt:

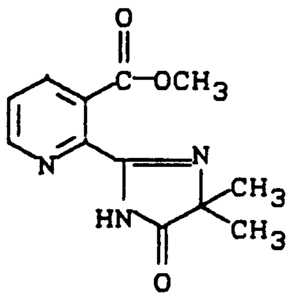

(A)

2-(4,4-Dimethylimidazolin-5-on-2-yl)-pyridin-3-carbonsäuremethylester (bekannt aus EP 41 623)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung
Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 1 und 3.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung
Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 1, 4, 7 und 10.

**Patentansprüche**

**1.** Substituierte Imidazolinylpyrimidine der allgemeinen Formel (I)

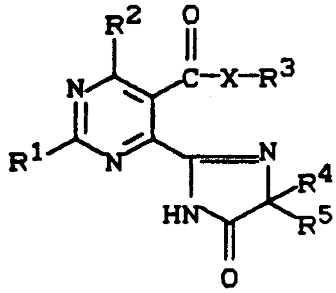

(I)

bei welchen

| | |
|---|---|
| $R^1$ | für Wasserstoff, Halogen, Cyano, Nitro oder Thiocyanato oder für einen jeweils gegebenenfalls substituierten Hydroxy-, Mercapto-, Amino-, Carbonyl-, Sulfinyl-, Sulfonyl-, Alkyl-, Alkenyl-, Alkinyl-, Cycloalkyl-, Cycloalkenyl-, Aryl- oder Heterocyclylrest steht, |
| $R^2$ | für Wasserstoff, Hydroxy, Halogen, Alkyl oder Halogenalkyl steht, |
| $R^3$ | für Wasserstoff, für jeweils gegebenenfalls substituiertes Alkyl, Alkenyl, Alkinyl, Cycloalkyl, Cycloalkenyl, Aryl oder Heterocyclyl oder für ein Äquivalent eines anorganischen oder organischen Kations steht, |
| $R^4$ und $R^5$ | unabhängig voneinander jeweils für Wasserstoff, Alkyl, Halogenalkyl oder Cycloalkyl stehen und |
| X | für Sauerstoff oder Schwefel steht. |

**2.** Substituierte Imidazolinylpyrimidine der allgemeinen Formel (I) gemäß Anspruch 1, bei welchen

$R^1$ für Wasserstoff, Fluor, Chlor, Brom, Iod, Cyano, Nitro oder Thiocyanato steht;

außerdem für jeweils gegebenenfalls substituiertes Hydroxy oder Mercapto steht, wobei als Substituenten jeweils infrage kommen: geradkettiges oder verzweigtes Alkyl mit 1 bis 8 Kohlenstoffatomen, geradkettiges oder verzweigtes Halogenalkyl mit 1 bis 8 Kohlenstoffatomen und 1 bis 17 gleichen oder verschiedenen Halogenatomen, geradkettiges oder verzweigtes Alkoxyalkyl mit jeweils 1 bis 8 Kohlenstoffatomen in den einzelnen Alkylteilen, geradkettiges oder verzweigtes Dialkylaminoalkyl mit jeweils 1 bis 8 Kohlenstoffatomen in den einzelnen Alkylteilen, geradkettiges oder verzweigtes Alkanoyl mit 1 bis 9 Kohlenstoffatomen, geradkettiges oder verzweigtes Alkoxycarbonyl mit 2 bis 9 Kohlenstoffatomen, geradkettiges oder verzweigtes Alkylsulfonyl mit 1 bis 8 Kohlenstoffatomen, geradkettiges oder verzweigtes Alkoxysulfonyl mit 1 bis 8 Kohlenstoffatomen, jeweils gegebenenfalls im Aryl-bzw. Heterocyclylteil einfach oder mehrfach, gleich oder verschieden substituiertes Arylalkyl mit 1 bis 8 Kohlenstoffatomen im geradkettigen oder verzweigten Alkylteil und 6 bis 10 Kohlenstoffatomen im Arylteil, Aryl mit 6 bis 10 Kohlenstoffatomen, Arylcarbonyl, Arylsulfonyl oder Aryloxysulfonyl mit jeweils 6 bis 10 Kohlenstoffatomen im Arylteil, Heterocyclyl oder Heterocyclylalkyl mit jeweils 2 bis 9 Kohlenstoffatomen und 1 bis 5 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

außerdem für gegebenenfalls einfach oder zweifach, gleich oder verschieden substituiertes Amino steht, wobei als Substituenten infrage kommen:

geradkettiges oder verzweigtes Alkyl mit 1 bis 8 Kohlenstoffatomen, geradkettiges

oder verzweigtes Alkenyl mit 2 bis 8 Kohlenstoffatomen, geradkettiges oder verzweigtes Alkinyl mit 2 bis 8 Kohlenstoffatomen, geradkettiges oder verzweigtes Halogenalkyl mit 1 bis 8 Kohlenstoffatomen und 1 bis 17 gleichen oder verschiedenen Halogenatomen, geradkettiges oder verzweigtes Alkoxyalkyl mit jeweils 1 bis 8 Kohlenstoffatomen in den einzelnen Alkylteilen geradkettiges oder verzweigtes Dialkylaminoalkyl mit jeweils 1 bis 8 Kohlenstoffatomen in den einzelnen Alkylteilen, geradkettiges oder verzweigtes Alkanoyl mit 1 bis 9 Kohlenstoffatomen, geradkettiges oder verzweigtes Alkoxycarbonyl mit 2 bis 9 Kohlenstoffatomen, geradkettiges oder verzweigtes Alkylsulfonyl mit 1 bis 8 Kohlenstoffatomen, geradkettiges oder verzweigtes Alkoxysulfonyl mit 1 bis 8 Kohlenstoffatomen, geradkettiges oder verzweigtes Dialkylaminosulfonyl mit jeweils 1 bis 8 Kohlenstoffatomen in den einzelnen Alkylteilen, jeweils gegebenenfalls im Aryl- bzw. Heterocyclylteil einfach oder mehrfach, gleich oder verschieden substituiertes Arylalkyl mit 1 bis 8 Kohlenstoffatomen im geradkettigen oder verzweigten Alkylteil und 6 bis 10 Kohlenstoffatomen im Arylteil, Aryl mit 6 bis 10 Kohlenstoffatomen, Arylcarbonyl, Arylsulfonyl oder Aryloxysulfonyl mit jeweils 6 bis 10 Kohlenstoffatomen im Arylteil, Heterocyclyl oder Heterocyclylalkyl mit jeweils 2 bis 9 Kohlenstoffatomen und 1 bis 5 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
außerdem für eine jeweils substituierte Carbonyl-, Sulfinyl- oder Sulfonylgruppe steht, wobei als Substituenten jeweils infrage kommen:
Wasserstoff, Hydroxy, Amino, jeweils geradkettiges oder verzweigtes Alkyl, Alkoxy, Alkylamino oder Dialkylamino mit jeweils 1 bis 8 Kohlenstoffatomen in den einzelnen Alkylteilen, jeweils geradkettiges oder verzweigtes Halogenalkyl oder Halogenalkoxy mit jeweils 1 bis 8 Kohlenstoffatomen und 1 bis 17 gleichen oder verschiedenen Halogenatomen, jeweils gegebenenfalls im Arylteil einfach oder mehrfach, gleich oder verschieden substituiertes Aryl, Aryloxy, Arylamino oder Diarylamino mit jeweils 6 bis 10 Kohlenstoffatomen in den einzelnen Arylteilen, wobei als Substituenten in den einzelnen Arylteilen jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
außerdem für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Fluor, Chlor, Brom, Iod, Hydroxy, Cyano, Nitro, Aminocarbonyl, Formamido, jeweils geradkettiges oder verzweigtes Alkoxy, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 8 Kohlenstoffatomen in den einzelnen Alkylteilen und gegebenenfalls 1 bis 17 gleichen oder verschiedenen Halogenatomen, jeweils gegebenenfalls im Aryl- oder Heterocyclylteil einfach oder mehrfach, gleich oder verschieden substituiertes Aryl, Aryloxy, Arylthio, Arylamino, Diarylamino, Arylcarbonyl oder Arylsulfonyl mit jeweils 6 bis 10 Kohlenstoffatomen in den einzelnen Arylteilen oder Heterocyclyl mit 2 bis 9 Kohlenstoffatomen und 1 bis 5

gleichen oder verschiedenen Heteroatomen, insbesondere Stickstoff, Sauerstoff und/oder Schwefel, wobei als Aryl- oder Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

außerdem für gegebenenfalls einfach oder mehrfach, gleich oder verschieden substituiertes, geradkettiges oder verzweigtes Alkenyl mit 2 bis 8 Kohlenstoffatomen steht, wobei als Substituenten infrage kommen:

Fluor, Chlor, Brom, Iod oder Cyano;

außerdem für gegebenenfalls einfach oder mehrfach, gleich oder verschieden substituiertes, geradkettiges oder verzweigtes Alkinyl mit 2 bis 8 Kohlenstoffatomen steht, wobei als Substituenten infrage kommen:

Fluor, Chlor, Brom oder Iod;

außerdem für jeweils gegebenenfalls einfach oder mehrfach, gleich oder verschieden substituiertes Cycloalkyl oder Cycloalkenyl mit jeweils 3 bis 8 Kohlenstoffatomen steht, wobei als Substituenten jeweils infrage kommen:

Fluor, Chlor, Brom, Iod sowie jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen;

außerdem für jeweils gegebenenfalls einfach oder mehrfach, gleich oder verschieden substituiertes Aryl mit 6 bis 10 Kohlenstoffatomen oder Heterocyclyl mit 2 bis 9 Kohlenstoffatomen und 1 bis 5 gleichen oder verschiedenen Heteroatomen, insbesondere Stickstoff, Sauerstoff und/oder Schwefel steht, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^2$ für Wasserstoff, Hydroxy, Fluor, Chlor, Brom, Iod, für geradkettiges oder verzweigtes Alkyl mit 1 bis 8 Kohlenstoffatomen oder für geradkettiges oder verzweigtes Halogenalkyl mit 1 bis 8 Kohlenstoffatomen und 1 bis 17 gleichen oder verschiedenen Halogenatomen steht,

$R^3$ für Wasserstoff oder für ein Äquivalent eines Alkali-, Erdalkali- oder eines gegebenenfalls einfach oder mehrfach, gleich oder verschieden durch geradkettiges oder verzweigtes Alkyl mit 1 bis 18 Kohstoffatomen und/oder Benzyl substituierten Ammoniumkations steht,

außerdem für gegebenenfalls einfach oder mehrfach, gleich oder verschieden substituiertes, geradkettiges oder verzweigtes Alkyl mit 1 bis 18 Kohlenstoffatomen steht, wobei als Substituenten infrage kommmen:

Fluor, Chlor, Brom, Iod, Hydroxy, Cyano, Nitro, Aminocarbonyl, Formamido, jeweils geradkettiges oder verzweigtes Alkoxy, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 8 Kohlenstoffatomen in den einzelnen Alkylteilen und gegebenenfalls 1 bis 17 gleichen oder verschiedenen Halogenatomen, jeweils gegebenenfalls im Aryl- oder Heterocyclylteil einfach oder mehrfach, gleich oder verschieden substituiertes Aryl, Aryloxy, Arylthio, Arylamino, Diarylamino, Arylcarbonyl oder Arylsulfonyl mit jeweils 6 bis 10 Kohlenstoffatomen in den einzelnen Arylteilen oder Heterocyclyl mit 2 bis 9 Kohlenstoffatomen und 1 bis 5

gleichen oder verschiedenen Heteroatomen, insbesondere Stickstoff, Sauerstoff und/oder Schwefel, wobei als Aryl- oder Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
außerdem für gegebenenfalls einfach oder mehrfach, gleich oder verschieden substituiertes, geradkettiges oder verzweigtes Alkenyl mit 2 bis 8 Kohlenstoffatomen steht, wobei als Substituenten infrage kommen: Fluor, Chlor, Brom, Iod, Cyano oder gegebenenfalls einfach oder mehrfach, gleich oder verschieden substituiertes Aryl mit 6 bis 10 Kohlenstoffatomen, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für gegebenenfalls einfach oder mehrfach, gleich oder verschieden substituiertes, geradkettiges oder verzweigtes Alkinyl mit 2 bis 8 Kohlenstoffatomen steht, wobei als Substituenten infrage kommen:
Fluor, Chlor, Brom oder Iod;
außerdem für jeweils gegebenenfalls einfach oder mehrfach, gleich oder verschieden substituiertes Cycloalkyl oder Cycloalkenyl mit jeweils 3 bis 8 Kohlenstoffatomen steht, wobei als Substituenten jeweils infrage kommen:
Fluor, Chlor, Brom, Iod sowie jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen;
außerdem für jeweils gegebenenfalls einfach oder mehrfach, gleich oder verschieden substituiertes Aryl mit 6 bis 10 Kohlenstoffatomen oder Heterocyclyl mit 2 bis 9 Kohlenstoffatomen und 1 bis 5 gleichen oder verschiedenen Heteroatomen, insbesondere Stickstoff, Sauerstoff und/oder Schwefel steht, wobei als Aryl- bzw. Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^4$ und $R^5$ unabhängig voneinander jeweils für Wasserstoff, für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 17 gleichen oder verschiedenen Halogenatomen oder für Cycloalkyl mit 3 bis 8 Kohlenstoffatomen stehen und

X für Sauerstoff oder Schwefel steht.

**3.** Substituierte Imidazolinylpyrimidine der allgemeinen Formel (I) gemäß Anspruch 1, bei welchen

$R^1$ für Wasserstoff, Fluor, Chlor, Brom, Iod, Cyano, Nitro oder Thiocyanato steht;
außerdem für jeweils gegebenenfalls substituiertes Hydroxy oder Mercapto steht, wobei als Substituenten jeweils infrage kommen: geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxyalkyl mit jeweils 1 bis 4 Kohlenstoffatomen in den einzelnen Alkylteilen, geradkettiges oder verzweigtes Dialkylaminoalkyl mit jeweils 1 bis 4 Kohlenstoffatomen in den einzelnen Alkylteilen, geradkettiges oder

verzweigtes Alkanoyl mit 1 bis 5 Kohlenstoffatomen, geradkettiges oder verzweigtes Alkoxycarbonyl mit 2 bis 5 Kohlenstoffatomen, geradkettiges oder verzweigtes Alkylsulfonyl mit 1 bis 4 Kohlenstoffatomen, geradkettiges oder verzweigtes Alkoxysulfonyl mit 1 bis 4 Kohlenstoffatomen, jeweils gegebenenfalls im Aryl- bzw. Heterocyclylteil ein-bis fünffach, gleich oder verschieden substituiertes Arylalkyl mit 1 bis 4 Kohlenstoffatomen im geradkettigen oder verzweigten Alkylteil und 6 oder 10 Kohlenstoffatomen im Arylteil, Aryl mit 6 oder 10 Kohlenstoffatomen, Arylcarbonyl, Arylsulfonyl oder Aryloxysulfonyl mit jeweils 6 oder 10 Kohlenstoffatomen im Arylteil, Heterocyclyl oder Heterocyclylalkyl mit jeweils 2 bis 9 Kohlenstoffatomen und 1 bis 4 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;

außerdem für gegebenenfalls einfach oder zweifach, gleich oder verschieden substituiertes Amino steht, wobei als Substituenten infrage kommen:

geradkettiges oder verzweigtes Alkyl mit 1 bis 4 Kohlenstoffatomen, geradkettiges oder verzweigtes Alkenyl mit 2 bis 5 Kohlenstoffatomen, geradkettiges oder verzweigtes Alkinyl mit 2 bis 5 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxyalkyl mit jeweils 1 bis 4 Kohlenstoffatomen in den einzelnen Alkylteilen, geradkettiges oder verzweigtes Dialkylaminoalkyl mit jeweils 1 bis 4 Kohlenstoffatomen in den einzelnen Alkylteilen, geradkettiges oder verzweigtes Alkanoyl mit 1 bis 5 Kohlenstoffatomen, geradkettiges oder verzweigtes Alkoxycarbonyl mit 2 bis 5 Kohlenstoffatomen, geradkettiges oder verzweigtes Alkylsulfonyl mit 1 bis 4 Kohlenstoffatomen, geradkettiges oder verzweigtes Alkoxysulfonyl mit 1 bis 4 Kohlenstoffatomen, geradkettiges oder verzweigtes Dialkylaminosulfonyl mit jeweils 1 bis 4 Kohlenstoffatomen in den einzelnen Alkylteilen, jeweils gegebenenfalls im Aryl-bzw. Heterocyclylteil ein- bis fünffach, gleich oder verschieden substituiertes Arylalkyl mit 1 bis 4 Kohlenstoffatomen im geradkettigen oder verzweigten Alkylteil und 6 oder 10 Kohlenstoffatomen im Arylteil, Aryl mit 6 oder 10 Kohlenstoffatomen, Arylcarbonyl, Arylsulfonyl oder Aryloxysulfonyl mit jeweils 6 oder 10 Kohlenstoffatomen im Arylteil, Heterocyclyl oder Heterocyclylalkyl mit jeweils 2 bis 9 Kohlenstoffatomen und 1 bis 4 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;

außerdem für eine jeweils substituierte Carbonyl-, Sulfinyl- oder Sulfonylgruppe steht, wobei als Substituenten jeweils infrage kommen:

Wasserstoff, Hydroxy, Amino, jeweils geradkettiges oder verzweigtes Alkyl, Alkoxy, Alkylamino oder Dialkylamino mit jeweils 1 bis 4 Kohlenstoffatomen in den einzelnen Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen

Halogenatomen, jeweils gegebenenfalls im Arylteil ein- bis fünffach, gleich oder verschieden substituiertes Aryl, Aryloxy, Arylamino oder Diarylamino mit jeweils 6 oder 10 Kohlenstoffatomen in den einzelnen Arylteilen, wobei als Substituenten in den einzelnen Arylteilen jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;
außerdem für gegebenenfalls einfach bis fünffach, gleich oder verschieden substituiertes, geradkettiges oder verzweigtes Alkyl mit 1 bis 6 Kohlenstoffatomen steht, wobei als Substituenten infrage kommen:
Fluor, Chlor, Brom, Iod, Hydroxy, Cyano, Nitro, Aminocarbonyl, Formamido, jeweils geradkettiges oder verzweigtes Alkoxy, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 4 Kohlenstoffatomen in den einzelnen Alkylteilen und gegebenenfalls 1 bis 9 gleichen oder verschiedenen Halogenatomen, jeweils gegebenenfalls im Aryl- oder Heterocyclylteil ein- bis fünffach, gleich oder verschieden substituiertes Aryl, Aryloxy, Arylthio, Arylamino, Diarylamino, Arylcarbonyl oder Arylsulfonyl mit jeweils 6 oder 10 Kohlenstoffatomen in den einzelnen Arylteilen oder Heterocyclyl mit 2 bis 9 Kohlenstoffatomen und 1 bis 4 gleichen oder verschiedenen Heteroatomen, insbesondere Stickstoff, Sauerstoff und/oder Schwefel, wobei als Aryl- oder Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;
außerdem für gegebenenfalls einfach bis dreifach, gleich oder verschieden substituiertes, geradkettiges oder verzweigtes Alkenyl mit 2 bis 5 Kohlenstoffatomen steht, wobei als Substituenten in Frage kommen:
Fluor, Chlor, Bro, Iod oder Cyano;
außerdem für gegebenenfalls einfach bis dreifach, gleich oder verschieden substituiertes, geradkettiges oder verzweigtes Alkinyl mit 2 bis 5 Kohlenstoffatomen steht, wobei als Substituenten infrage kommen:
Fluor, Chlor, Brom oder Iod;
außerdem für jeweils gegebenenfalls einfach bis fünffach, gleich oder verschieden substituiertes Cycloalkyl oder Cycloalkenyl mit jeweils 3 bis 6 Kohlenstoffatomen steht, wobei als Substituenten jeweils infrage kommen: Fluor, Chlor, Brom, Iod sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen;
außerdem für jeweils gegebenenfalls einfach bis fünffach, gleich oder verschieden substituiertes Aryl mit 6 oder 10 Kohlenstoffatomen oder Heterocyclyl mit 2 bis 9 Kohlenstoffatomen und 1 bis 4 gleichen oder verschiedenen Heteroatomen, insbesondere Stickstoff, Sauerstoff und/oder Schwefel steht, wobei als Arylbzw. Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;

$R^2$ für Wasserstoff, Hydroxy, Fluor, Chlor, Brom, Iod, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^3$ für Wasserstoff oder für ein Äquivalent eines Alkali-, Erdalkali- oder eines gegebenenfalls einfach bis vierfach, gleich oder verschieden durch geradkettiges oder verzweigtes Alkyl mit 1 bis 18 Kohlenstoffatomen und/oder Benzyl substituierten Ammoniumkations steht, außerdem für gegebenenfalls einfach bis fünffach, gleich oder verschieden substituiertes, geradkettiges oder verzweigtes Alkyl mit 1 bis 12 Kohlenstoffatomen steht, wobei als Substituenten infrage kommen:

Fluor, Chlor, Brom, Iod, Hydroxy, Cyano, Nitro, Aminocarbonyl, Formamido, jeweils geradkettiges oder verzweigtes Alkoxy, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 8 Kohlenstoffatomen in den einzelnen Alkylteilen und gegebenenfalls 1 bis 17 gleichen oder verschiedenen Halogenatomen, jeweils gegebenenfalls im Aryl- oder Heterocyclylteil einfach bis fünffach, gleich oder verschieden substituiertes Aryl, Aryloxy, Arylthio, Arylamino, Diarylamino, Arylcarbonyl oder Arylsulfonyl mit jeweils 6 oder 10 Kohlenstoffatomen in den einzelnen Arylteilen oder Heterocyclyl mit 2 bis 9 Kohlenstoffatomen und 1 bis 4 gleichen oder verschiedenen Heteroatomen, insbesondere Stickstoff, Sauerstoff und/oder Schwefel, wobei als Aryl- oder Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;

außerdem für gegebenenfalls einfach bis dreifach, gleich oder verschieden substituiertes, geradkettiges oder verzweigtes Alkenyl mit 2 bis 5 Kohlenstoffatomen steht, wobei als Substituenten infrage kommen:

Fluor, Chlor, Brom, Iod, Cyano oder gegebenenfalls ein- bis dreifach, gleich oder verschieden substituiertes Aryl mit 6 oder 10 Kohlenstoffatomen, wobei als Substituenten infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;

außerdem für gegebenenfalls einfach bis dreifach, gleich oder verschieden substituiertes, geradkettiges oder verzweigtes Alkinyl mit 2 bis 5 Kohlenstoffatomen steht, wobei als Substituenten infrage kommen:

Fluor, Chlor, Brom oder Iod;

außerdem für jeweils gegebenenfalls einfach bis fünffach, gleich oder verschieden substituiertes Cycloalkyl oder Cycloalkenyl mit jeweils 3 bis 6 Kohlenstoffatomen steht, wobei als Substituenten jeweils infrage kommen:

Fluor, Chlor, Brom, Iod sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen;

außerdem für jeweils gegebenenfalls einfach bis fünffach, gleich oder verschieden substituiertes Aryl mit 6 oder 10 Kohlenstoffatomen oder Heterocyclyl mit 2 bis 9 Kohlenstoffatomen und 1 bis 4 gleichen oder verschiedenen Heteroatomen, insbesondere Stickstoff, Sauerstoff und/oder Schwefel steht, wobei als Aryl- bzw. Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls ein- bis fünffach, gleich oder verschieden durch Halogen und/oder geradkettiges oder verzweigtes Alkyl mit 1 bis 4 Kohlenstoffatomen substituiertes Phenyl;

$R^4$ und $R^5$ unabhängig voneinander jeweils für Wasserstoff, für geradkettiges oder verzweigtes Alkyl mit 1 bis 4 Kohlenstoffatomen, für geradkettiges oder verzweigtes Halogenalkyl mit 1 bis 4 Kohlenstoffatomen und 1 bis 9 gleichen oder verschiedenen Halogenatomen oder für Cycloalkyl mit 3 bis 6 Kohlenstoffatomen stehen und

X für Sauerstoff oder Schwefel steht.

**4.** Substituierte Imidazolinylpyrimidine der allgemeinen Formel (I) gemäß Anspruch 1, bei welchen

$R^1$ für Wasserstoff steht;
außerdem für jeweils gegebenenfalls substituiertes Hydroxy oder Mercapto steht, wobei als Substituenten jeweils infrage kommen:
geradkettiges oder verzweigtes Alkyl mit 1 bis 4 Kohlenstoffatomen, geradkettiges oder verzweigtes Alkoxyalkyl mit jeweils 1 bis 4 Kohlenstoffatomen in den einzelnen Alkylteilen, geradkettiges oder verzweigtes Dialkylaminoalkyl mit jeweils 1 bis 4 Kohlenstoffatomen in den einzelnen Alkylteilen, geradkettiges oder verzweigtes Halogenalkyl mit 1 bis 4 Kohlenstoffatomen und 1 bis 9 gleichen oder verschiedenen Halogenatomen oder jeweils gegebenenfalls im Phenyl- bzw. Heteroarylteil ein- bis dreifach, gleich oder verschieden substituiertes Phenylalkyl mit 1 bis 4 Kohlenstoffatomen im geradkettigen oder verzweigten Alkylteil, Phenyl, Pyridyl, Pyridylmethyl, Furanylmethyl, Thienylmethyl, Thiazolylmethyl oder Triazolylmethyl, wobei als Phenyl- bzw. Heteroarylsubstituenten jeweils infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylatomen steht, wobei als Substituenten infrage kommen:
Fluor, Chlor, jeweils geradkettiges oder verzweigtes Alkoxy oder Alkylthio mit jeweils 1 bis 4 Kohlenstoffatomen, jeweils geradkettiges oder verzweigtes Halogenalkoxy oder Halogenalkylthio mit jeweils 1 bis 4 Kohlenstoffatomen und 1 bis 9 Halogenatomen oder gegebenenfalls ein- bis dreifach, gleich oder verschieden substituiertes Phenyl, wobei als Substituenten infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für geradkettiges oder verzweigtes Alkenyl mit 3 bis 6 Kohlenstoffatomen steht;
außerdem für geradkettiges oder verzweigtes Alkinyl mit 2 bis 6 Kohlenstoffatomen steht;
außerdem für gegebenenfalls ein- bis dreifach, gleich oder verschieden durch Methyl und/oder Chlor substituiertes Cyclopropyl steht;
außerdem für gegebenenfalls ein- bis dreifach, gleich oder verschieden substituiertes Phenyl steht, wobei als Substituenten jeweils infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für jeweils gegebenenfalls ein- bis dreifach, gleich oder verschieden substituiertes Pyridyl, Furanyl, Tetrahydrofuranyl, Thienyl oder Thiazolyl steht, wobei als Substituenten jeweils infrage kommen:
jeweils geradkettiges oder verzweigtes Alkyl oder Alkoxy mit jeweils 1 bis 4 Kohlenstoffatomen;

$R^2$ für Wasserstoff oder Methyl steht,

$R^3$ für Wasserstoff oder für ein Äquivalent eines Alkali-, Erdalkali- oder eines gegebenenfalls einfach bis vierfach, gleich oder verschieden durch geradkettiges oder verzweigtes Alkyl mit

1 bis 18 Kohlenstoffatomen und/oder Benzyl substituierten Ammoniumkations steht;
außerdem für gegebenenfalls ein- bis dreifach, gleich oder verschieden substituiertes, geradkettiges oder verzweigtes Alkyl mit 1 bis 12 Kohlenstoffatomen steht, wobei als Substituenten infrage kommen:
Fluor, Chlor, Hydroxy, Cyano, jeweils geradkettiges oder verzweigtes Alkoxy, Alkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 8 Kohlenstoffatomen in den einzelnen Alkylteilen, jeweils geradkettiges oder verzweigtes Halogenalkoxy oder Halogenalkylthio mit jeweils 1 bis 8 Kohlenstoffatomen und 1 bis 17 Halogenatomen sowie jeweils gegebenenfalls im Phenyl- oder Heteroarylteil einfach bis dreifach, gleich oder verschieden substituiertes Phenyl, Phenoxy, Phenylthio, Phenylamino, Phenylcarbonyl, Phenylsulfonyl, Pyridyl, Furanyl, Tetrahydrofuranyl, Thienyl oder Thiazolyl, wobei als Phenyl- oder Heteroarylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen;
außerdem für geradkettiges oder verzweigtes Alkenyl mit 2 bis 6 Kohlenstoffatomen steht;
außerdem für geradkettiges oder verzweigtes Alkinyl mit 2 bis 6 Kohlenstoffatomen steht;
außerdem für jeweils gegebenenfalls ein-bis dreifach, gleich oder verschieden durch Fluor, Chlor und/oder Methyl substituiertes Cyclopentyl oder Cyclohexyl steht;
außerdem für gegebenenfalls ein- bis dreifach, gleich oder verschieden substituiertes Phenyl steht, wobei als Substituenten jeweils infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für jeweils gegebenenfalls ein- bis dreifach, gleich oder verschieden substituiertes Pyridyl, Furanyl, Tetrahydrofuranyl, Thienyl oder Thiazolyl steht, wobei als Substituenten jeweils infrage kommen:
jeweils geradkettiges oder verzweigtes Alkyl oder Alkoxy mit jeweils 1 bis 4 Kohlenstoffatomen;

$R^4$ für Wasserstoff, Methyl oder Fluormethyl steht,
$R^5$ für Methyl, Ethyl, n- oder i-Propyl oder Cyclopropyl steht und
X für Sauerstoff oder Schwefel steht.

**5.** Substituierte Imidazolinylpyrimidine der allgemeinen Formel (I) gemäß Anspruch 1, bei welchen

$R^1$ für Wasserstoff, Hydroxy, Methoxy, Ethoxy, n-oder i-Propoxy, n-, i-, s- oder t-Butoxy, Trifluormethoxy, Mercapto, Methylthio, Ethylthio, n-oder i-Propylthio, n-, i-, s-oder t-Butylthio, Trifluormethylthio, Benzylthio, 2-Pyridylthio, Amino, N-Methylamino, N-Ethylamino, N-n-Propylamino, N-i-Propylamino, N,N-Dimethylamino, N,N-Diethylamino, N-Methyl-N-Ethylamino, Methylcarbonylamino, Ethylcarbonylamino, Methoxycarbonylamino, Ethoxycarbonylamino, Methylsulfinyl, Ethylsulfinyl, Methylsulfonyl, Ethylsulfonyl, Methyl, Ethyl, n- oder i-Propyl, n-, i-, s-oder t-Butyl, n-oder i-Pentyl, n- oder i-Hexyl, Fluormethyl, Trifluormethyl, Methoxymethyl, Ethoxymethyl, Methylthiomethyl, Ethylthiomethyl, Allyl, Ethinyl, Propargyl, Cyclopropyl oder für gegebenenfalls einfach oder zweifach, gleich oder verschieden substituiertes Phenyl steht, wobei als Substituenten infrage kommen:
Fluor, Chlor, Brom, Methyl, Ethyl, n- oder i-Propyl, n-, i-, s- oder t-Butyl, Methoxy, Ethoxy, n-oder i-Propoxy, n-, i-, s- oder t-Butoxy, Methylthio, Ethylthio, Trifluormethyl, Trifluormethoxy oder Trifluormethylthio;
außerdem für jeweils gegebenenfalls einfach oder zweifach, gleich oder verschieden substituiertes Pyridyl, Furanyl, Thienyl oder Thiazolyl steht, wobei als Substituenten jeweils infrage kommen:
Methyl, Ethyl, n- oder i-Propyl, n-, i-, s-oder t-Butyl, Methoxy, Ethoxy, n- oder i-Propoxy, n-, i-, s- oder t-Butoxy,

$R^2$ für Wasserstoff steht,
$R^3$ für Wasserstoff, für ein Natrium-, Kalium-, Calcium- oder für ein gegebenenfalls ein- bis vierfach, gleich oder verschieden durch Methyl, Ethyl, n- oder i-Propyl, n-, i-, s-oder t-Butyl,

n- oder i-Pentyl, n- oder i-Hexyl, n- oder i- Dodecyl und/ oder Benzyl substituiertes Ammoniumion steht;
außerdem für Methyl, Ethyl, n- oder i-Propyl, n-, i-, s- oder t-Butyl, n- oder i-Pentyl, n-oder i-Hexyl, Fluormethyl, Trifluormethyl, Hydroxyethyl, Cyanoethyl, Methoxyethyl, Methylthioethyl, Methylaminoethyl, Ethylaminoethyl, N,N-Dimethylaminoethyl, N,N-Diethylaminoethyl, Methylcarbonylmethyl, Methylcarbonylethyl, N-Methylaminocarbonylmethyl, N-Methylaminocarbonylethyl, N-Ethylaminocarbonylmethyl, N-Ethylaminocarbonylethyl, N,N-Dimethylaminocarbonylmethyl, N,N-Diethylaminocarbonylmethyl, N,N-Dimethylaminocarbonylethyl, N,N-Diethylaminocarbonylethyl, N-Acetylaminomethyl, N-Acetylaminoethyl, N-Propionylaminomethyl, N-Propionylaminoethyl, Methylsulfonylmethyl, Methylsulfonylethyl, Ethylsulfonylmethyl, Ethylsulfonylethyl, Allyl, Propargyl, Cyclopentyl, Cyclohexyl odr für jeweils gegebenenfalls ein- bis dreifach, gleich oder verschieden substituiertes Phenyl, Pyridyl, Benzyl oder Furanylmethyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio,

$R^4$ für Methyl steht,
$R^5$ für Isopropyl steht und
X für Sauerstoff steht.

**6.** Verfahren zur Herstellung von substituierten Imidazolinylpyrimidinen der allgemeinen Formel (I) gemäß Anspruch 1

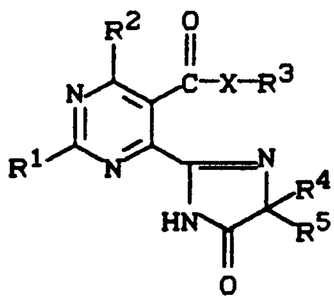

(I)

bei welchen

$R^1$ für Wasserstoff, Halogen, Cyano, Nitro oder Thiocyanato oder für einen jeweils gegebenenfalls substituierten Hydroxy-, Mercapto-, Amino-, Carbonyl-, Sulfinyl-, Sulfonyl-, Alkyl-, Alkenyl-, Alkinyl-, Cycloalkyl-, Cycloalkenyl-, Aryl- oder Heterocyclylrest steht,
$R^2$ für Wasserstoff, Hydroxy, Halogen, Alkyl oder Halogenalkyl steht,
$R^3$ für Wasserstoff, für jeweils gegebenenfalls substituiertes Alkyl, Alkenyl, Alkinyl, Cycloalkyl, Cycloalkenyl, Aryl oder Heterocyclyl oder für ein Äquivalent eines anorganischen oder organischen Kations steht,
$R^4$ und $R^5$ unabhängig voneinander jeweils für Wasserstoff, Alkyl, Halogenalkyl oder Cycloalkyl stehen und
X für Sauerstoff oder Schwefel steht,

dadurch gekennzeichnet, daß man
(a) Pyrimidin-4,5-dicarbonsäurediester der Formel

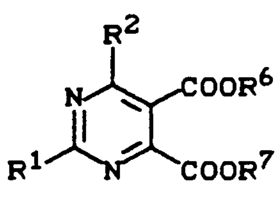

(II)

in welcher

$R^1$ und $R^2$ die oben angegebene Bedeutung haben und
$R^6$ und $R^7$ unabhängig voneinander jeweils für Alkyl stehen,
mit α-Aminosäureamiden der Formel (III),

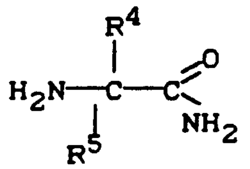

(III)

in welcher
$R^4$ und $R^5$ die oben angegebene Bedeutung haben, gegebenenfalls in Gegenwart eines Verdünnungsmittels und gegebenenfalls in Gegenwart eines Reaktionshilfsmittels umsetzt, oder daß man
(b) in einer anschließenden Folgereaktion die so erhältlichen Imidazolinylpyrimidincarbonsäuren der Formel (Ia),

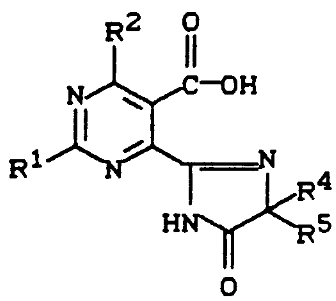

(Ia)

in welcher
$R^1$, $R^2$, $R^4$ und $R^5$ die oben angegebene Bedeutung haben,
zunächst in einer 1. Stufe mit einem Kondensationsmittel gegebenenfalls in Gegenwart eines Verdünnungsmittels und gegebenenfalls in Gegenwart eines Reaktionshilfsmittels umsetzt und anschließend die so erhältlichen Imidazo-pyrrolo-pyrimidine der Formeln (IVa) und (IVb),

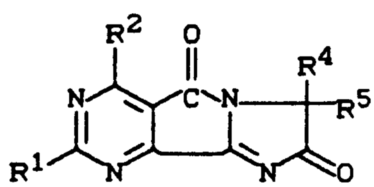

(IVa)

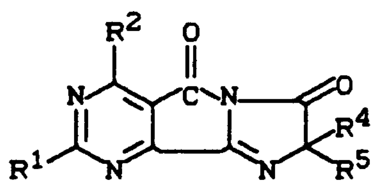

(IVb)

in welcher
$R^1$, $R^2$, $R^4$ und $R^5$ die oben angegebene Bedeutung haben,
in einer anschließenden 2. Stufe mit Alkoholen oder Thiolen der Formel (V),

$R^3—X—H$ (V)

in welcher

$R^3$ und X die oben angegebene Bedeutung haben,

gegebenenfalls in Gegenwart eines Verdünnungsmittels und gegebenenfalls in Gegenwart eines Reaktionshilfsmittels umsetzt.

**7.** Herbizide Mittel, gekennzeichnet durch einen Gehalt an mindestens einem substituierten Imidazolinylpyrimidin der Formel (I) gemäß den Ansprüchen 1 bis 6.

**8.** Verfahren zur Bekämpfung von unerwünschten Pflanzen, dadurch gekennzeichnet, daß man substituierte Imidazolinylpyrimidine der Formel (I) gemäß den Ansprüchen 1 bis 6 auf unerwünschte Pflanzen und/oder ihren Lebensraum einwirken läßt.

**9.** Verwendung von substituierten Imidazolinylpyrimidinen der Formel (I) gemäß den Ansprüchen 1 bis 6 zur Bekämpfung von unerwünschten Pflanzen.

**10.** Verfahren zur Herstellung von herbiziden Mitteln, dadurch gekennzeichnet, daß man substituierte Imidazolinylpyrimidine der Formel (I) gemäß den Ansprüchen 1 bis 6 mit Streckmitteln und/oder oberflächenaktiven Substanzen vermischt.

**11.** Substituierte Imidazopyrrolopyrimidine der allgemeinen Formeln (IVa) und (IVb)

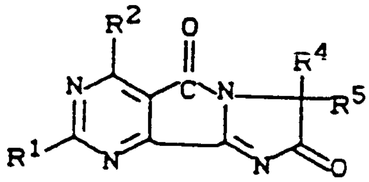

(IVa)

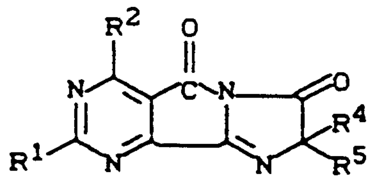

(IVb)

in welcher

$R^1$ für Wasserstoff, Halogen, Cyano, Nitro oder Thiocyanato oder für einen jeweils gegebenenfalls substituierten Hydroxy-, Mercapto-, Amino-, Carbonyl-, Sulfinyl-, Sulfonyl-, Alkyl-, Alkenyl-, Alkinyl-, Cycloalkyl-, Cycloalkenyl-, Aryl- oder Heterocyclylrest steht,

$R^2$ für Wasserstoff, Hydroxy, Halogen, Alkyl oder Halogenalkyl steht,

$R^4$ und $R^5$ unabhängig voneinander jeweils für Wasserstoff, Alkyl, Halogenalkyl oder Cycloalkyl stehen.

Europäisches Patentamt

# EUROPÄISCHER RECHERCHENBERICHT

Nummer der Anmeldung

EP 92 10 8733

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 215 738 (CIBA-GEIGY)<br>* das ganze Dokument *<br>--- | 1,6-11 | C07D403/04<br>C07D401/14<br>C07D487/14 |
| A | EP-A-0 202 654 (SQUIBB & SONS)<br>* Seite 17 - Seite 34; Ansprüche *<br>----- | 1,6-11 | C07D415/14<br>A01N43/54 |

RECHERCHIERTE SACHGEBIETE (Int. Cl.5)

C07D

Der vorliegende Recherchenbericht wurde für alle Patentansprüche erstellt

| Recherchenort | Abschlußdatum der Recherche | Prüfer |
|---|---|---|
| DEN HAAG | 14 SEPTEMBER 1992 | FRANCOIS J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)